# EUROPÄISCHE PATENTSCHRIFT

(11) **EP 2 377 484 B1**
(45) Veröffentlichungstag und Bekanntmachung des Hinweises auf die Patenterteilung: **19.08.2015**
(21) Anmeldenummer: 10011069.1
(22) Anmeldetag: 08.06.2006
(51) Int. Cl.: A61B 19/00

(54) **Vorrichtung und Verfahren zum berührungslosen Ermitteln und Vermessen einer Raumposition und/oder einer Raumorientierung von Körpern**
Device and method for contactless determination and measuring of the spatial position and/or spatial orientation of bodies
Dispositif et procédé d'établissement et de mesure sans contact d'une positon spatiale et/ou d'une orientation spatiale de corps

(30) Priorität: 09.06.2005 DE 102005026654; 28.11.2005 DE 102005056897; 29.11.2005 DE 102005057237; 23.12.2005 DE 102005062384
(43) Veröffentlichungstag der Anmeldung: 19.10.2011
(62) Teilanmeldung aus: 06754233.2
(73) Patentinhaber: Naviswiss AG, 4242 Laufen (CH)
(72) Erfinder: Knobel, Bruno, 4242 Laufen (CH); Findeisen, Charles, 5430 Wettingen (CH); Keeve, Erwin, 14469 Potsdam (DE); Widmer, Karl-Heinz, 79249 Merzhausen (DE); Bartl, Frank, 79395 Neuenburg (DE); Hämmerle, Christoph, 8121 Benglen (CH)
(74) Vertreter: Castell, Klaus

(56) Entgegenhaltungen:
- EP-A2- 0 672 389
- WO-A1-2005/032390
- WO-A1-2005/076033
- US-A- 5 676 673

## Beschreibung

Die Erfindung betrifft sowohl eine Vorrichtung als auch ein Verfahren zum berührungslosen Ermitteln und Vermessen einer Raumposition und/oder einer Raumorientierung von Körpern, mit Körpern wie einem medizinischen Werkzeug und/oder einem medizinischen Instrument und mit einem Trackingsystem, mittels welchem die Körper lokalisiert und zueinander in Relation gebracht werden.

Vorrichtungen und Verfahren zum berührungslosen Ermitteln und Vermessen einer Raumposition und/oder einer Raumorientierung von Körpern sind insbesondere bei computerassistierten Eingriffen in der Chirurgie aus dem Stand der Technik bekannt. Hierbei werden oft Navigationssysteme verwendet, welche zumindest teilweise auf optischen Trackingsystemen basieren. Bei den bekannten Trackingsystemen wird eine Raumlage, das heißt, insbesondere die Raumposition und/oder die Raumorientierung, von mit beispielsweise Lokatoren ausgestatteten Instrumenten und Körperteilen mit bekannten mathematischen Verfahren in Echtzeit ermittelt.

Beispielsweise ist aus der Offenlegungsschrift DE 196 39 615 A1 ein Neuronavigationssystem bekannt, welches ein Reflektorenreferenzierungssystem mit passiven Reflektoren und mit einem Markersystem, welches Marker oder Landmarken umfasst, aufweist. Die Reflektoren und die Marker sind an den zu behandelnden Körperteilen und an den chirurgischen Instrumenten vorgesehen, so dass ihre Lokalisation mittels einer Rechner-/Kameraeinheit problemlos möglich ist. Die Raumlagen bzw. Daten hierzu können an einer Grafik-Bildschirmausgabe angezeigt werden.

Die Rechner-/Kameraeinheit wird gegenüber den zu behandelnden Körperteilen eines Patienten stationär aufgestellt. Unter Zuhilfenahme weiterer Daten, die beispielsweise durch eine Computertomografie ermittelt wurden, wird die interessierende Patientenanatomie näher erfasst. Unterstützend wirken hierbei an dem Patienten angebrachte Reflektoren bzw. Marker, mittels welcher die Raumlage des Patienten erfasst wird. Anhand der hierbei gewonnenen Daten können Raumlagen von verwendeten chirurgischen Instrumenten gegenüber dem Patienten verfolgt und gegebenenfalls korrigiert werden. Das im Stand der Technik beschriebene Neuronavigationssystem ermöglicht eine gute und zielgenaue Führung der chirurgischen Instrumente. Nachteilig ist jedoch der relativ groß bauende apparative Aufwand, der während einer OP sich störend auswirken kann. Dies trifft insbesondere bei räumlich eher beengten Verhältnissen zu.

Eine gattungsgemäße Vorrichtung ist aus der WO 2005/076033 A1 bekannt. Hier ist das Trackingsystem an einem stationären Ständer angeordnet, der nur eine begrenzte Schwenk-Neige-Bewegung ermöglicht.

Weitere ähnliche Vorrichtungen sind aus der US 5 676 673 A, der WO 2005/032390 A1 und der EP 0 672 389 A2 bekannt.

Es ist Aufgabe vorliegender Erfindung bekannte Trackingsysteme weiter zu entwickeln, so dass sich deren Handhabung wesentlich vereinfacht.

Die Aufgabe der Erfindung wird von einer Vorrichtung zum berührungslosen Ermitteln und Vermessen einer Raumposition und/oder einer Raumorientierung von Körpern mit einem Trackingsystem, mittels welchem die Körper lokalisiert und zueinander in Relation gebracht werden, gelöst, die die Merkmale des Patentanspruchs 1 aufweist.

Alle bisher bekannten Trackingsysteme sind stationär in einem gewissen Abstand vom Operationsfeld fest auf stabilen Ständern montiert und so ortsfest gegenüber den zu erfassenden Körpern fixiert. Sie dürfen dabei während der Messungen nicht angestoßen werden, um die Messergebnisse nicht negativ zu beeinträchtigen. Dies führt insbesondere bei beengten Platzverhältnissen zu Problemen, so dass die bekannten Trackingsysteme trotz aller Vorzüge auch hinderlich sein können. Dies liegt auch daran, dass an zu erfassenden Körpern verwendete Lokatoren bei der Messung stets von dem Trackingsystem einsehbar sein müssen, aber leicht von einem Operateur oder weiterem Personal verdeckt werden können.

Die Einschränkung, dass das Trackingsystem während seines Betriebs bzw. während seiner Verwendung stationär fixiert sein muss, ist bei vorliegendem mobilen Trackingsystem erfindungsgemäß nicht erforderlich.

Der Begriff "stationär" beschreibt in vorliegendem Zusammenhang ein Trackingsystem, welches insbesondere während seiner Verwendung ortfest gelagert ist, welches also während seiner Verwendung nicht bewegt wird. Die Verwendung des Trackingsystems ist hierbei im eigentlichen "Tracken" eines Körpers zu sehen. Es versteht sich, dass ein herkömmliches Trackingsystem auch umpositionierbar ist. Ein derartiges Umpositionieren geschieht aber nicht während des Trackens sondern, falls erforderlich, nur vor oder nach einem Tracken. Hierdurch sind die Trackingsysteme aus dem Stand der Technik insbesondere während des Trackens nicht im Sinne des vorliegenden Trackingsystems mobil einsetzbar.

Somit unterscheidet sich das vorliegende mobile Trackingsystem wesentlich von bekannten Trackingsystemen.

Die Bezeichnung "Trackingsystem" beschreibt vorliegend eine Einrichtung, mittels welcher ein Körper, insbesondere ein medizinisches Werkzeug, ein medizinisches Werkstück, ein medizinisches Instrument und/oder ein medizinisches Hilfsmittel optisch ermittelt und eine Relativbewegung zwischen diesem und einem weiteren Körper verfolgt werden kann. Insbesondere ist mit dem Begriff "Trackingsystem" eine zu dem Trackingsystem gehörige Optik und Sensorik sowie ein diesbezüglicher apparativer Aufbau erfasst, so dass zumindest Bauteile oder Bauteilgruppen des Trackingsystems, welche die Optik bzw. Sensorik umfassen, gegenüber den zu trackenden Körpern mobil verlagerbar sind.

Der Begriff "Körper" erfasst im Sinne der Patentanmeldung sowohl allgemeine technische Gebilde als auch spezielle technische Gebilde auf dem Gebiet der Medizin, wie etwa medizinische Werkzeuge, medizinische Werkstücke, medizinische Instrumente, Implantate oder sonstige technische Hilfsmittel, die in der Medizin zur Anwendung gelangen.

Körper, wie beispielsweise medizinische Werkzeuge, medizinische Werkstücke, medizinische Instrumente und/oder Implantate können optional mit austauschbaren und/oder verstellbaren sowie in geeigneter Kombination mit Strukturen und Lokatoren ausgerüsteten Einsätzen bestückt sein.

Unter dem Begriff "technische Hilfsmittel" versteht man vorliegend im Besonderen Hilfsmittel, die zur Bestimmung einer relativen Raumlage von Körpern zueinander verwendet werden. Technische Hilfsmittel im Zusammenhang mit Trackingsystemen auf dem Gebiet der Medizin sind beispielsweise Lokatoren, Strukturen, Landmarken und Muster an Körpern.

Mit den Begriffen "Raumposition" und "Raumorientierung" wird im Sinne der vorliegenden Patentanmeldung eine Raumlage eines Körpers, beispielsweise im Koordinatensystem des Trackingsystems, und/oder eine relative Raumlage zweier Körper zueinander beschrieben.

Die Aufgabe der Erfindung wird auch von einem Verfahren zum berührungslosen Ermitteln und Vermessen einer Raumposition und/oder einer Raumorientierung von Körpern mit einem Trackingsystem, mittels welchem die Körper lokalisiert und zueinander in Relation gebracht werden, gelöst, das die Merkmale des Patentanspruchs 12 aufweist.

Erfindungsgemäß kann vorliegend das Trackingsystem selbst während seiner Verwendung, also während des "Tracking" von Körpern bewegt, gegenüber den Körpern verlagert und sogar temporär bei Seite gelegt werden. Somit sind insbesondere die eingangs erwähnten Nachteile bekannter Trackingsysteme vorliegend beseitigt.

Sowohl die erfindungsgemäße Vorrichtung als auch das erfindungsgemäße Verfahren eignen sich sehr gut dazu, Trackingsysteme und Trackingverfahren auf dem Gebiet der Medizin weiter zu entwickeln und zu verbessern. Es versteht sich, dass sich die vorliegende Vorrichtung und das vorliegende Verfahren jedoch nicht nur auf den Anwendungsbereich der Medizin beschränkt, sondern überall dort vorteilhaft eingesetzt werden können, wo eine Bestimmung von Raumlagen mindestens zweier Körper vorteilhaft und/oder erforderlich ist.

Eine bevorzugte Ausführungsvariante sieht vor, dass das mobile Trackingsystem während seines Betriebs bzw. während seiner Verwendung lose gegenüber den Körpern gehalten ist.

Insbesondere hierdurch unterscheidet sich das Trackingsystem der vorliegenden Vorrichtung von herkömmlichen Trackingsystemen. Herkömmliche Trackingsysteme müssen gegenüber den zu erfassenden Körpern immer stationär angeordnet sein oder zumindest einen Bezug zu einem Raumkoordinatensystem besitzen.

Vorteilhafter Weise kann ein Anwender, beispielsweise ein Operateur, das vorliegende Trackingsystem bei Seite legen, falls dieses in einer Arbeitsphase nicht benötigt wird. Hierdurch wird der Einsatz des Trackingsystems sehr flexibel.

Besonders vorteilhaft ist es deshalb, wenn das mobile Trackingsystem während seines Betriebs bzw. während seiner Verwendung verlagerbar gegenüber den Körpern angeordnet und/oder gehalten ist. Hierdurch kann das Trackingsystem der Vorrichtung während einer Arbeit zeitlich nur dann und örtlich nur dort eingesetzt werden, wenn es erforderlich ist. Andernfalls wird das Trackingsystem bei Seite gelegt.

So sieht eine bevorzugte Ausführungsvariante vor, dass das mobile Trackingsystem gegenüber dem Körper ortslose angeordnet ist.

Der Begriff "ortslose" beschreibt im vorliegenden Zusammenhang einen Zustand, der genau das Gegenteil von der Bedeutung "ortsfest" kennzeichnet. Somit ist das Trackingsystem vorteilhafter Weise nicht starr gegenüber einem gesamten Arbeitsbereich oder einem lokalen Arbeitsgebiet fixiert. Vielmehr wird das mobile Trackingsystem selbst während eines operativen Eingriffs gegenüber den zu erfassenden Körpern verlagert und bewegt.

Eine diesbezügliche Verfahrensvariante sieht vor, dass das Trackingsystem während seiner Verwendung händisch geführt wird. Hierbei kann beispielsweise ein Operateur selbst entscheiden, wo und wann das Trackingsystem vor einem lokalen Arbeitsgebiet gehalten wird.

Das mobile Trackingsystem ist einfach zu bedienen, wenn es während seiner Verwendung, insbesondere händisch, unmittelbar vor die zu erfassenden lokalen Arbeitsgebiete gehalten wird.

Alle bekannten Trackingsysteme erfassen immer nahezu das gesamte Arbeitsgebiet mit der geforderten hohen Messgenauigkeit, wodurch sich ein enormer Rechenaufwand ergibt. Vorteilhafter Weise reduziert sich vorliegend zudem der Rechenaufwand zur Bestimmung der Raumposition und/oder der Raumorientierung von Körpern, da nicht wie bisher üblich, der gesamte Arbeitsbereich von dem vorliegenden Trackingsystem erfasst wird, sondern nur das lokale Arbeitsgebiet, welches beispielsweise für den operativen Eingriff unmittelbar von Interesse ist.

Eine weitere Verfahrensvariante sieht vor, dass zumindest die Raumposition und/oder die Raumorientierung zwischen wenigstens zwei Körpern lediglich phasenweise ermittelt werden. Hierdurch reduziert sich der erforderliche Rechenaufwand weiter.

Unter dem Begriff "phasenweise" versteht man vorliegend, dass das mobile Trackingsystem nicht permanent vor einem Arbeitsgebiet angeordnet ist. Beispielsweise wird das mobile Trackingsystem hinsichtlich einer Vorher-/Nachher-Betrachtung nur vor einem Haupteingriff und nach dem Haupteingriff vor das lokale Arbeitsgebiet gehalten. So kann das mobile Trackingsystem zwischenzeitlich bei Seite gelegt werden.

Somit kann das vorliegende Trackingsystem für die Messung oder die Kontrolle vorteilhafter Weise lediglich vor und nach bestimmten Arbeitsschritten eingesetzt werden. Dazu wird es in vorteilhafter Position und Messrichtung auf die beispielsweise mit Lokatoren oder Strukturen ausgestatteten Objekte ausgerichtet. Das Trackingsystem kann die Raumlage der Objekte als Momentaufnahme relativ zueinander erfassen. Das Trackingsystem kann auch die Raumlage der Objekte mittels einer Aufnahmesequenz aufzeichnen, um beispielsweise das dynamische Verhalten von Körperteilen vor, während oder nach gewissen Arbeitsschritten zu studieren. Diese Vorgehensweise hat den wesentlichen Vorteil gegenüber stationären Systemen, dass nicht mehr das gesamte Arbeitsvolumen mit der verlangten Genauigkeit dauernd eingesehen werden muss sondern nur noch das eigentliche lokale Arbeitsgebiet mit den durchzuführenden oder durchgeführten Arbeitsschritten. Ein weiterer Vorteil ist, dass die Position und die Messrichtung des Trackingsystems für jeden Arbeitsschritt optimal ausgewählt werden können. Besonders für nahe beieinander liegende Objekte bzw. Körper ist es vorteilhaft, dass äußerst handliche Trackingsysteme mit kleinen Abmessungen verwendet werden können.

Um das mobile Trackingsystem von Hand gut führen zu können, ist es vorteilhaft, wenn das mobile Trackingsystem ein Griffteil zum händischen Halten und/oder zum Führen des mobilen Trackingsystems aufweist.

Eine weitere Erleichterung in der Handhabung ergibt sich, wenn das mobile Trackingsystem ein Gewicht von weniger als 2 kg oder von weniger als 0,5 kg, vorzugsweise von weniger als 0,1 kg, aufweist. Mittels eines derart geringen Gewichtes ist das mobile Trackingsystem selbst bei einem längeren Einsatz gut zu handhaben. Insbesondere mobile Trackingsysteme sind mit einem Gewicht von weniger als 0,1 kg besonders vorteilhaft in der Dentalmedizin und/oder der minimalinvasiven Medizin einsetzbar.

Um das Trackingsystem nur im Bedarfsfall zu aktivieren, ist es vorteilhaft, wenn das mobile Trackingsystem eine Auslöseeinrichtung zum Starten und Ausführen einer Ermittlung und einer Vermessung aufweist.

Verfahrenstechnisch ist es demnach vorteilhaft, wenn ein Ermitteln und/oder ein Vermessen mittels einer manuellen Auslöseeinrichtung eingeleitet wird.

Es versteht sich, dass die Auslöseeinrichtung vielfältig gestaltet sein kann. Beispielsweise befindet sich die Auslöseeinrichtung in Form eines Schalters am Griffteil. Eine Ausführungsvariante sieht vor, dass die Auslöseeinrichtung einen Fußschalter umfasst. Bei dieser Ausgestaltung kann das mobile Trackingsystem noch kompakter gebaut werden, da auf Bauteile oder Bauteilgruppen hinsichtlich eines Schalters unmittelbar am mobilen Trackingsystem verzichtet werden kann.

Eine weitere Ausführungsvariante sieht vor, dass die Auslöseeinrichtung Mittel zur Sprachsteuerung aufweist. Hierdurch kann ein Operateur das mobile Trackingsystem besonders komfortabel steuern.

Für den Fall, dass das mobile Trackingsystem nicht ausschließlich von Hand gehalten und geführt wird, ist es vorteilhaft, wenn das mobile Trackingsystem eine Befestigungseinrichtung zum Anordnen an einer verlagerbaren Führungseinrichtung, wie einem manuell verlagerbaren Haltearm und/oder einem Roboterarm, aufweist. Mittels einer derartigen Befestigungseinrichtung ist das Anbringen des mobilen Trackingsystems an zusätzliche Hilfshalteeinrichtungen, wie beispielsweise an einem während des Trackens verlagerbaren Haltearms oder wie an einem Roboterarm, wesentlich vereinfacht.

Die Befestigungseinrichtung kann auch derart ausgestaltet sein, dass sie dazu geeignet ist, dass mobile Trackingsystem an einem Arm eines Operateurs anzuordnen. Es kann vorteilhaft sein, wenn die Befestigungseinrichtung es ermöglicht, das mobile Trackingsystem unmittelbar am Kopf oder einem sonstigen Körperteil eines Patienten zu befestigen.

Für eine weitere verbesserte Handhabung des mobilen Trackingsystems ist es darüber hinaus vorteilhaft, wenn das mobile Trackingsystem autark ist.

Um das Trackingsystem technisch immer auf dem neusten Stand der Technik halten zu können, ist es vorteilhaft, wenn die Berechnungsmittel als Software ausgeführt sind. Eine Software ist besonders günstig upzudaten.

Zum autarken Betrieb des mobilen Trackingsystems ist es vorteilhaft, wenn das mobile Trackingsystem eine eigene Energiequelle aufweist. Beispielsweise ist die Energiequelle als Batterie, Akkumulator oder Brennstoffzelle ausgebildet.

Die Handhabung des mobilen Trackingsystems ist weiter erleichtert, wenn das mobile Trackingsystem Mittel zur drahtlosen Kommunikation aufweist.

Der Begriff "Lokatoren" beschreibt vorliegend insbesondere ein technisches Hilfsmittel, welches an einem weiteren Körper anordenbar ist. Der weitere Körper ist mittels eines Lokators von dem mobilen Trackingsystem lokalisierbar. Anhand des Lokators ist die Raumlage eines Körpers problemlos bestimmbar.

Mit dem Begriff "Struktur" wird im Sinne der Patentanmeldung ein dreidimensionales Gebilde beschrieben, welches ein Muster aufweist. Eine Struktur bildet vorliegend auch einen Körper im Sinne der Patentanmeldung. Die Struktur ist beispielsweise als ebene Platte ausgebildet. Die Struktur kann permanent oder abnehmbar an einem weiteren Körper angeordnet sein. In einigen Anwendungsfällen kann es von Vorteil sein, wenn die Struktur an sich ein Körperteil eines weiteren Körpers bildet. Beispielsweise ist die Struktur ein Teilbereich eines medizinischen Instrumentes.

Vorzugsweise ist das Muster auf der Oberfläche der Struktur aufgebracht. Es kann aber auch in anderer Weise an der Struktur angeordnet sein. Die Gestalt eines Strukturmusters wird bereits bei der Herstellung der Struktur festgelegt.

Ein "Muster" ist im Sinne der Patentanmeldung ein Gebilde, welches aus geraden oder gekrümmten Linien unterschiedlicher Breite und Länge, aus Kreisen, aus Ellipsen, aus Dreiecken und aus Rechtecken oder aus Kombinationen daraus besteht. Es ist vorzugsweise direkt auf eine Oberfläche eines Körpers, wie einer Struktur, aufgebracht. Wesentlich ist es hierbei, dass das Muster kontrastreich von einer Oberfläche unterscheidbar ist, auf welche es angebracht ist.

Das Muster unterscheidet sich etwa von einer Struktur dadurch, dass es im Wesentlichen ein zweidimensionales Gebilde und kein dreidimensionales Gebilde ist. Das bedeutet, dass das Muster als Gebilde wie etwa eine Struktur zwar eine Länge und eine Breite aufweist. Jedoch ist die Dicke des Musters gegenüber der Dicke einer Struktur derart vernachlässigbar gering, dass im vorliegenden Zusammenhang ein Muster ein zweidimensionalen Gebilde ist. Bei einem Muster reduziert sich die Dicke beispielsweise auf eine auf einer Struktur aufgebrachten Farbschicht. Ein Muster kann auch aus einer Kombination aus reflektierenden, absorbierenden, fluoreszierenden, phosphoreszierenden oder selbstleuchtenden Materialien bestehen. Diese Materialien eignen sich besonders im Zusammenhang mit für den Menschen sichtbarem Licht, infrarotem oder ultraviolettem Licht.

Ein Teilbereich eines Musters kann dazu dienen, eine Algorithmuseffizienz eines mobilen Trackingsystems zu steigern. Ist ein Muster auf einer Struktur aufgebracht, kann das Muster oder ein Teilbereich davon zur Identifikation der Struktur dienen. Mittels des Musters kann insbesondere definiert werden, ob es sich bei einem der Struktur weiter zugeordneten Körper um ein medizinisches Instrument oder um ein medizinisches Werkzeug handelt. Das Muster liefert somit wesentliche Informationen von mittels des Trackingsystems ermittelten Körpern an das mobile Trackingsystem.

Neben dem beschriebenen Muster existieren im Sinne der Patentanmeldung als eine weitere Mustergattung "sekundäre Muster". Insbesondere für eine Registrierung von Körpern ist es vorteilhaft, wenn neben den vom mobilen Trackingsystem ermittelten Muster noch weitere Muster, die sekundären Muster, existieren, welche auch von anderen bildgebenden Systemen, etwa von einem CT-Gerät oder von einer sonstigen Vorrichtung zur dreidimensionalen Erfassung von Knochen und Zähnen mittels Röntgenstrahlung, erkannt und gemessen werden können.

Vorliegend ist also die Rede von einem Muster, wenn dieses vorwiegend nur von dem mobilen Trackingsystem ermittelt wird. Ist ein Muster von einem weiteren bildgebenden System ermittelbar und räumlich auflösbar, spricht man vorliegend von einem sekundären Muster. Das sekundäre Muster besteht deshalb vorzugsweise aus röntgen-opaken und/oder röntgen-transparenten Materialien. Sekundäre Muster können zudem Materialien umfassen, welche von MRI-Systemen erkannt und gemessen werden können. In einem weiteren Beispiel bestehen sekundäre Muster aus Materialien, die von bildgebenden Systemen erkannt und gemessen werden können, welche auf dem Terahertz-Spektralbereich basieren.

Je nach Art des bildgebenden Systems, wie eines CT- oder eines MRI-Systems, sollten die sekundären Muster aus geraden oder gekrümmten Linien unterschiedlicher Breite und Länge, aus Kreisen, aus Ellipsen, aus Dreiecken und aus Rechtecken oder aus Kombinationen daraus bestehen, welche eine kritische Schichtdicke nicht unterschreiten dürfen.

Sind beispielsweise Körperteile, wie Knochen-/Gelenkteile, einer Person mit Lokatoren, Strukturen, Muster und/oder sekundären Muster versehen, können diese ebenfalls zumindest von dem mobilen Trackingsystem erkannt werden.

Darüber hinaus ist es vorteilhaft, wenn ein Muster für das mobile Trackingsystem erkennbare und verwertbare gerade oder gekrümmte Linien unterschiedlicher Breite und Länge, Kreise, Ellipsen, Dreiecke und Rechtecke oder aus Kombinationen daraus umfasst.

Des Weiteren ist es vorteilhaft, wenn ein Muster Licht nicht reflektierende Mittel, Licht reflektierende Mittel, fluoreszierende Mittel, phosphoreszierende Mittel, und/oder weitere selbstleuchtende Mittel umfasst.

Eine Ausführungsvariante sieht vor, dass ein sekundäres Muster in einem definierten geometrischen Bezug zu einem Muster steht. Insbesondere für eine Registrierung ist es vorteilhaft, dass es neben dem vom mobilen Trackingsystem gemessenen Muster noch ein wohl definiertes, sekundäres Muster gibt. Das sekundäre Muster aus geeigneten Materialien wird auch von anderen bildgebenden Systemen erkannt und gemessen. So kann das sekundäre Muster von den CT-Geräten oder sonstigen Vorrichtungen zur dreidimensionalen Erfassung von Knochen und Zähnen mittels Röntgenstrahlung erkannt und räumlich aufgelöst werden, wenn das sekundäre Muster aus der Kombination von röntgen-opaken und röntgen-transparenten Materialien besteht.

Vorteilhaft ist es also, wenn ein sekundäres Muster von weiteren bildgebenden, insbesondere nicht optischen, Systemen erkennbar ist.

Darüber hinaus ist es vorteilhaft, wenn eine Struktur Muster aufweist, deren Lage und/oder deren geometrische Details bekannt sind. Hierdurch kann das mobile Trackingsystem einen die Muster aufweisenden Körper exakt vermessen.

Um Strukturen an Körpern vorteilhaft befestigen zu können, ist es vorteilhaft, wenn eine Struktur Mittel zum Fixieren an Körpern aufweist.

In diesem Zusammenhang ist es vorteilhaft, wenn die Fixiermittel Abstützfüße zum Fixieren an Körpern aufweisen.

Auch ist es vorteilhaft, wenn eine Struktur eine ebene Platte umfasst. Insbesondere eine ebene Platte kann gut mit einem Muster versehen werden.

Eine diesbezügliche Ausführungsvariante sieht vor, dass eine Struktur eine ebene Platte mit einer dreidimensional strukturierten Oberfläche umfasst.

Baulich besonders vorteilhaft ist es, wenn eine Struktur ein Teil eines medizinischen Werkzeuges, eines medizinischen Werkstückes und/oder eines medizinischen Instrumentes bildet. Hierdurch lässt sich die Struktur vorteilhaft in einen Körper integrieren.

Eine weitere vorteilhafte Befestigungsmöglichkeit ergibt sich, wenn eine Struktur einen Magnet umfasst.

Damit eine Struktur von dem mobilen Trackingsystem immer gut erkennbar ist, ist es vorteilhaft, wenn eine Struktur eine schmutzabweisende Oberfläche aufweist.

Um zu trackende Körper, insbesondere Muster auf Körpern oder Strukturen an Körpern, besonders gut erkennen zu können, ist es vorteilhaft, wenn das mobile Trackingsystem Mittel zum Beleuchten aufweist.

Je nach Anwendungsgebiet, ist es vorteilhaft, wenn die Beleuchtungsmittel Leuchtmittel für den hyperroten Bereich aufweisen.

Es ist vorteilhaft, wenn ein Körper deformierbare Elemente umfasst. Mittels der deformierbaren Elemente ist ein Körper vielseitig einstellbar und kann an vorgefundene Gegebenheiten angepasst werden.

Um beispielsweise Deformationen an einem Körper, wie beispielsweise an einem medizinischen Werkzeug oder an einem medizinischen Instrument, feststellen zu können, ist es besonders vorteilhaft, wenn eine Geometrie eines Körpers ermittelt und vermessen wird. Hierbei spielt es keine Rolle, ob es sich bei den Körperdeformationen um beabsichtigte oder unbeabsichtigte Deformationen handelt.

Es ist weiterhin vorteilhaft, wenn die Körper mittels auf und/oder an den Körpern befindlichen Mustern und/oder einem sekundären Mustern, welche von weiteren bildgebenden Systemen erkannt werden, registriert werden.

Eine weitere Vereinfachung ergibt sich, wenn das mobile Trackingsystem an einem Körper, insbesondere an einem medizinischen Werkzeug und/oder an einem medizinischen Instrument, angeordnet wird. Beispielsweise ist das mobile Trackingsystem an einem Körperteil, wie einem Knochen, eines Patienten temporär fixiert.

Vorteilhaft ist es, wenn die Raumposition und/oder die Raumorientierung von an Körpern angeordneten Lokatoren relativ zu an den Körpern angeordneten Strukturen und/oder Mustern ermittelt und vermessen werden.

Um Muster an Strukturen mittels des mobilen Trackingsystems erkennen zu können, welche nicht unmittelbar im Sichtbereich des mobilen Trackingsystems liegen, ist es vorteilhaft, wenn die Vorrichtung einen Spiegel umfasst, welcher Muster und/oder sekundäre Muster aufweist. Mittels des Spiegels können die nicht unmittelbar einsehbaren Muster zumindest mittelbar für das mobile Trackingsystem einsehbar werden. Dadurch, dass an dem Spiegel ebenfalls Muster und/oder sekundäre Muster angeordnet sind, ist die Raumlage des Spiegels insbesondere mittels des mobilen Trackingsystems bestimmbar. Durch die Raumlagedaten des Spiegels kann dann die Raumlage der mittels des Spiegels einsehbaren Muster ermittelt werden.

Mit einem Verfahren zum Kalibrieren von insbesondere medizinischen Werkzeugen, von medizinischen Werkstücken und/oder von medizinischen Instrumenten, bei welchem die medizinischen Werkzeuge, die medizinischen Werkstücke und/oder die medizinischen Instrumente mit Mustern und/oder mit Strukturen, welche Muster umfassen, versehen werden und die medizinischen Werkzeuge, die medizinischen Werkstücke und/oder die medizinischen Instrumente mittels der Mustern und/oder der Strukturen vermessen werden reduziert sich der Aufwand zum Kalibrieren derartiger Körper wesentlich.

Die geforderten mechanischen Genauigkeiten insbesondere hinsichtlich medizinischer Instrumente bedingen teilweise sehr hohe Herstellungskosten. Dies gilt insbesondere für medizinische Instrumente, mit austauschbaren oder verstellbaren Einsätzen, deren Spitzen beispielsweise mit Skalpellklingen oder Tastspitzen versehen sind.

Wird das medizinische Instrument ohne vorheriges Kalibrieren eingesetzt, ergeben sich die erreichbaren Genauigkeiten der Raumlagen unter anderem aus der Toleranz der Instrumentenfertigung. Zusätzlich besteht immer die Möglichkeit, dass versehentlich deformierte Instrumente eingesetzt werden. Mit zusätzlichen Kalibriervorrichtungen werden im Stand der Technik deshalb die Geometrien der medizinischen Instrumente in der Regel vor oder zwischen bestimmten Arbeitsphasen mit dem optischen Trackingsystem ausgemessen und geprüft. Auf diese zusätzlichen Kalibriervorrichtungen kann vorliegend vorteilhafter Weise verzichtet werden.

Vorteilhaft ist es somit, wenn die Muster und/oder die Strukturen mittels eines Trackingsystems, insbesondere eines mobilen Trackingsystems, ermittelt werden, um die medizinischen Instrumente zu vermessen.

Ein Kalibrierverfahren mit einer Vorrichtung, bei dem insbesondere das mobile Trackingsystem die Struktur der Instrumente oder der Werkzeuge misst, hat den wesentlichen Vorteil, dass die üblichen aufwendigen Kalibriervorrichtungen entfallen oder vereinfacht werden können.

Die Form der Struktur und die Eigenschaften des Musters werden bereits bei der Herstellung definiert. Aus der Kenntnis des Musters sind damit auch die wesentlichen geometrischen Gegebenheiten der Struktur bekannt. Wesentliche geometrische Gegebenheiten sind beispielsweise Durchmesser, Länge oder Radius. Ist beispielsweise die gesamte Oberfläche des nicht deformierten Objekts mit einem geeigneten Muster bedeckt, kann aus Kenntnis des Musters die geometrische Form des Objekts mit einer Messung ausreichend exakt beschrieben werden.

Die räumliche Lage der Lokatoren bezüglich den Strukturen wird beim Kalibrierverfahren vom Trackingsystem gemessen. Damit ist es nicht mehr notwendig, dass die räumliche Lage der Lokatoren bei der Herstellung aufwendig realisiert werden muss. Ein typischer Kalibriervorgang eines Instruments mit Struktur und Lokatoren kann folgendermaßen ablaufen.

Das Instrument wird im Messvolumen bzw. Arbeitsgebiet des Trackingsystems gedreht, so dass alle Seiten des Instruments im Kalibriermodus sequentiell gemessen werden. Alternativ kann das Trackingsystem selber um das Instrument geführt werden. Der Kalibriermodus beinhaltet des Weiteren die Erkennung und Auswertung der Strukturen, die Bestimmung der Geometrie der Struktur, die Zuordnung der Struktur bezüglich der räumlichen Gestalt des Instruments auf Grund der Identifikation sowie die räumliche Lage von Lokatoren bezüglich der Struktur respektive zur räumlichen Gestalt des Instruments.

Dieser Kalibriervorgang für ein Instrument oder ein Werkzeug kann nach Bedarf wiederholt werden. Da bei der Messung der Objektraumlagen vor, während oder nach einem Arbeitsgang typischerweise sowohl die Struktur (ganz oder teilweise) als auch die Lokatoren erfasst werden, kann die Kalibrierung gleichzeitig dauernd oder periodisch überprüft werden. Das Trackingsystem beinhaltet zur Erfüllung dieser Aufgaben entsprechend geeignete Algorithmen.

Bei einem Verfahren zum Prüfen von insbesondere medizinischen Werkzeugen, von medizinischen Werkstücken und/oder von medizinischen Instrumenten hinsichtlich vorhandener Deformationen können mittels eines mobilen Trackingsystems Muster auf den medizinischen Werkzeugen, den medizinischen Werkstücken und/oder auf den medizinischen Instrumenten ermittelt werden.

Es ist vorteilhaft, wenn Muster auf insbesondere medizinischen Werkzeugen, auf medizinischen Werkstücken und/oder auf medizinischen Instrumenten zum Kalibrieren und/oder zum Prüfen von Deformationen der medizinischen Werkzeuge, der medizinischen Werkstücke und/oder der medizinischen Instrumente verwendet werden.

Das mobile Trackingsystem ist insbesondere in der Lage, deformierte Instrumente zu messen und zu erkennen, da die aktuelle Struktur von der in der Identifikation definierten oder der vorgängig gemessenen Struktur abweicht. Die Deformation kann unabsichtlich beispielsweise schon vor dem Arbeitseinsatz oder während einem Arbeitsgang erfolgen. Die Deformation kann auch absichtlich durchgeführt werden, um beispielsweise die geometrische Form eines Instruments für einen Arbeitsschritt anzupassen. Insbesondere auch mittels des Kalibrierungsverfahrens kann die geometrische Form des deformierten Instruments ausreichend genau bestimmt werden.

Darüber hinaus unterstützt das mobile Trackingsystem, insbesondere in der Chirurgie, den Anwender wesentlich bei der Registrierung der einzelnen Körper, was eine erhebliche Vereinfachung eines herkömmlichen Registriervorgangs darstellt.

Diese Vereinfachung wird vorteilhafter Weise dadurch erreicht, dass einerseits das Trackingsystem eine Struktur und/oder ein Muster am entsprechenden Körperteil und andererseits die anderen bildgebenden Systeme (C-Bogen, CT, MRI und/oder einer sonstigen Vorrichtung zur dreidimensionalen Erfassung von Knochen und Zähnen mittels Röntgenstrahlung) die Patientendaten und die sekundären Muster erkennen und als Raumlageinformation aufbereiten. Dadurch ist jederzeit die Raumlage der Struktur bezüglich den Patientendaten bekannt. Damit entfallen die zeitintensiven präoperativen Bestimmungen der Registrierpunkte sowie das Abtasten dieser Punkte mit einem vom Trackingsystem gemessenen Zeigeinstrument. Das mobile Trackingsystem beinhaltet zur Erfüllung dieser Aufgabe vorzugsweise entsprechend optimierte Algorithmen. Dies hat den weiteren wesentlichen Vorteil, dass das taktile Verfahren für die Registrierung idealerweise nicht mehr erforderlich ist. Oder das taktile Verfahren wird gegebenenfalls noch zu Kontrollzwecken eingesetzt.

Es können auch Muster und/oder sekundäre Muster an wenigstens einem geeigneten Zahn oder an einer Schablone, welche an wenigstens einem geeigneten Zahn oder an einer an einem Kiefer befestigten Struktur angeordnet wird, angeordnet werden, bei welchem mittels der vorliegend erläuterten Vorrichtung während einer ersten Messung die relative Raumposition und/oder die relative Raumorientierung der Muster und/oder der sekundären Muster zueinander ermittelt werden.

Die vorliegende Erfindung ist somit auch im Bereich der Dentalmedizin besonders vorteilhaft.

Um weitere Daten über das Operationsgebiet zu erhalten, ist es vorteilhaft, wenn zuvor eine Oberfläche eines lokalen Arbeitsgebietes optisch gescannt wird.

Weitere Daten des Operationsgebietes können ermittelt werden, wenn das lokale Arbeitsgebiet dreidimensional mittels Röntgenstrahlung gemessen wird.

Ein Operateur erhält von dem Operationsgebiet ein sehr genaues Bild, wenn mittels ermittelter Scandaten und ermittelter Röntgendaten sowie ermittelter Daten eines mobilen Trackingsystems der Vorrichtung ein dreidimensionales Modell erstellt wird.

Vorzugsweise werden zum Erstellen des Modells zumindest die ermittelten Scandaten und die ermittelten Röntgendaten in ein gemeinsames Koordinatensystem übertragen.

Eine bevorzugte Verfahrensvariante sieht vor, dass mittels dieses Modells eine Konfiguration eines Implantats und/oder einer Krone ermittelt wird.

Ein Eingriff kann besonders präzise vorgenommen werden, wenn mittels dieses Modells eine relative Raumposition und/oder eine relative Raumorientierung eines Bohrers, einer Bohrachse, einer Bohrtiefe und/oder einer Ansetzposition ermittelt wird.

Weiter ist es vorteilhaft, wenn die ermittelte Konfiguration der Krone mittels Unterstützung von CAD/CAM im Wesentlichen zeitgleich zum chirurgischen Bohrvorgang gefertigt wird.

Zusammenfassend sei nochmals erwähnt, dass die die Erfindung betreffenden Trackingsysteme besonders gut geeignet sind, an den Objekten angebrachte Lokatoren und Strukturen zu erkennen, die Raumlagen der Lokatoren und Strukturen zu bestimmen und daraus die Raumlagen der Objekte zu ermitteln.

Das Trackingsystem ist darüber hinaus in der Lage, die ganze oder einen Teil der geometrischen Form von mit geeigneten Strukturen versehenen Objekten zu ermitteln. Des Weiteren setzt es die geometrische Form der Objekte in Bezug zu den am Objekt angebrachten Lokatoren.

Eine Objekt-Raumlage wird entweder mit den Lokatoren oder mit der Struktur ermittelt. Die Bestimmung der Objekt-Raumlage kann auch mit den Lokatoren und der Struktur erfolgen.

Das vorliegende mobile Trackingsystem kann auf einem Objekt bzw. Körper, wie auf einem Werkzeug, einem Instrument oder einem weiteren Körperteil, direkt angebracht sein. Dies hat den wesentlichen Vorteil, dass damit die Raumlage dieses Körpers ohne weitere Messung allein mittels des Trackingsystems bekannt ist.

Ein weiterer Vorteil ist darin zu sehen, dass sich die Anzahl der benötigten Körper im Arbeitsbereich reduziert. Im Extremfall befindet sich in der Anwendung beispielsweise nur noch das mit dem mobilen Trackingsystem ausgerüstete Werkzeug, welches sich beispielsweise an einer an einem Körperteil befestigten Struktur orientiert. Als besonders wichtiges Beispiel sei die Anwendung in der Dentalchirurgie erwähnt, bei dem das mobile Trackingsystem vorteilhafter Weise in bzw. an ein Arbeitsinstrument, wie beispielsweise einem Bohrgerät, integriert ist. Die Strukturen können hierbei auf einer Schablone und/oder auf Zähnen angebracht sein. Die Schablonen erhalten einen besonders sicheren Halt, wenn sie beispielsweise über einen oder mehrere Zähne gestülpt sind.

Das mobil, leicht, tragbar und handlich ausgebildete vorliegende Trackingsystem kann manuell nach Bedarf zur Messung eingesetzt werden. Es können damit die Raumlagen von Körpern vor und nach bestimmten Arbeitsschritten ausgemessen werden. Während der Arbeitsschritte kann es vorteilhafter Weise bei Seite gelegt werden. Das Trackingsystem kann somit, wie bereits erwähnt, auch auf einem Roboter, insbesondere an einem Roboterarm, montiert sein und hierbei nur bedarfsweise Messungen vornehmen.

Das vorliegende mobile Trackingsystem könnte zumindest temporär auch auf einem Stativ für eine Langzeitmessung der Objektraumlagen im Arbeitsbereich montiert sein. Wichtige Eigenschaften des mobilen Trackingsystems, wie das Messvolumen, der Bereich der Messdistanzen oder die Messgenauigkeit, werden unter anderen von der Anordnung und Eigenschaften der Kameras, der Lokatoren oder der Strukturen definiert. Die Messgenauigkeit, das Messvolumen und/oder der Bereich der Messdistanzen beeinflussen wesentlich die Geometrie des mobilen Trackingsystems und die Anordnung und Eigenschaften der Kameras. Kleine Messvolumen und kleine Bereiche der Messdistanzen erlauben typischerweise Trackingsysteme, welche kleine geometrische Dimensionen aufweisen. Umgekehrt haben große Messvolumen und große Bereiche der Messdistanzen typischerweise geometrisch größere Trackingsysteme zur Folge. Für gleiche Messgenauigkeiten und bei gleicher Kameratechnologie ist damit der Aufwand bei größeren Trackingsystemen bedeutend höher als bei kleineren Trackingsystemen.

An dieser Stelle sei darauf hingewiesen, dass die gesamten Instrumente oder Werkzeuge, Einsätze, die Strukturen, die Lokatoren oder Teile davon vorteilhafter Weise für einen einmaligen Gebrauch gefertigt sind. Dies ist bei bestimmten Anwendungen z.B. aus hygienischen Gründen vorteilhaft. Weitere Vorteile ergeben sich für die Logistik, Reinigung, Sterilisierung und Überprüfung der Körper.

Ein erheblicher Vorteil ist es weiter, dass solche Einwegteile mit geeigneten Materialien und mit geeigneten Fertigungsmethoden, wie beispielsweise Spritzguss oder plastisch formgebenden Techniken, kostengünstig hergestellt werden können.

Ein weiterer Vorteil liegt darin, dass die Fertigungsgenauigkeit der Lokatoren samt Befestigung reduziert sein kann, da die Lokatorenposition bezüglich der Struktur vorliegend mit dem mobilen Trackingsystem gemessen wird.

Ein Verfahren sieht vor, dass an einem Körper, wie etwa an einem Werkstück, eine erste Struktur mit Mustern und/oder ein erster Lokator und wenigstens eine weitere Struktur mit Mustern und/oder wenigstens ein weiterer Lokator angeordnet wird, bei welchem mittels der der Erfindung zugrunde liegenden Vorrichtung während einer ersten Messung die relative Raumposition und/oder die relative Raumorientierung der Strukturen mit den Mustern und/oder der Lokatoren zueinander ermittelt werden, bei welchem anschließend der Körper bearbeitet wird, bei welchem während wenigstens einer weiteren Messung erneut eine relative Raumposition und/oder eine relative Raumorientierung der Strukturen mit den Mustern und/oder der Lokatoren zueinander ermittelt werden, und bei welchem die ermittelten Raumpositionen und/oder die Raumorientierungen aus den beiden Messungen miteinander verglichen werden.

Vorteilhafter Weise kann hierbei besonders präzise ein Körper oder Teilbereiche eines Körpers, insbesondere hinsichtlich einer Raumlage, vor und nach einem Arbeitsvorgang verglichen werden.

Eine weiterführende Verfahrensvariante sieht vorteilhafter Weise vor, dass der bearbeitete Körper schrittweise derart weiter bearbeitet wird, bis die Raumposition und/oder die Raumorientierung der Strukturen mit den Mustern und/oder der Lokatoren aus der ersten Messung mit der Raumposition und/oder der Raumorientierung der Strukturen mit den Mustern und/oder der Lokatoren aus der zweiten Messung in geeigneter Weise übereinstimmen. Vorzugsweise werden die Raumposition und/oder die Raumorientierung lediglich anhand der vorhandenen Muster mittels des mobilen Trackingsystems ermittelt.

Hierdurch kann der Körper oder Teile des Körpers nach einem erfolgten Arbeitsvorgang mit höchster Präzision wieder an den Ausgangszustand angepasst werden.

Eine im medizinischen Bereich bevorzugte Verfahrensvariante sieht vor, dass ein Operateur einen Gelenk oder ein sonstiges Körperteil eines Patienten bearbeitet.

Wird beispielsweise ein Teil eines Gelenkes eines Patienten durch eine Prothese ersetzt oder ein Knochen derart bearbeitet, dass zwischen zwei Knochenteilen ein Implantat eingesetzt wird, ist es vorteilhaft, wenn vor dem Beginn des eigentlichen Haupteingriffes wenigstens zwei Strukturen mit Mustern geeignet an Seiten des Gelenkes befestigt werden und die relative Raumlage der Strukturen zueinander anhand der Muster mittels des mobilen Trackingsystems ermittelt wird. Zwischen den beiden Strukturen ersetzt der Operateur ein Gelenkteil durch eine Prothese. Nach erfolgtem Haupteingriff wird die Raumlage der Strukturen erneut ermittelt und mit der zuvor ermittelten Raumlage verglichen. Somit hat der Operateur unmittelbar nach dem Haupteingriff die nachweisliche Sicherheit, dass das Gelenk oder Teile davon wieder in seiner Ursprunglage angeordnet ist bzw. sind oder dieser Ursprunglage in einem medizinisch ausreichenden Maße sehr nahe kommen.

Auf Grund der besonders einfachen Handhabung ist das vorliegende Trackingsystem besonders vorteilhaft im Bereich der Dentalmedizin und der minimalinvasiven Medizin einsetzbar.

Weitere Vorteile, Ziele und Eigenschaften vorliegender Erfindung werden anhand nachfolgender Erläuterung anliegender Zeichnung beschrieben, in welcher beispielhaft Vorrichtungen zum berührungslosen Ermitteln und Vermessen einer Raumposition und/oder einer Raumorientierung von Körpern mit einem Trackingsystem sowie Bauteile oder Bauteilgruppen derartiger Vorrichtungen dargestellt sind.

Es zeigt
- Figur 1: schematisch eine Anordnung der erfindungsgemäßen Vorrichtung mit einem mobilen Trackingsystem mit einem medizinischen Instrument, einschließlich einer Struktur mit einem Muster vor einem Körperteil mit einer weiteren Struktur,
- Figur 2: schematisch ein Ausführungsbeispiel eines ersten Musters,
- Figur 3: schematisch ein Ausführungsbeispiel eines weiteren Musters,
- Figur 4: schematisch ein weiteres Musterbeispiel,
- Figur 5: schematisch eine Anordnung aus einem mobilen Trackingsystem und einem medizinischen Instrument mit Lokatoren, Strukturen und Mustern,
- Figur 6: schematisch eine Ansicht einer Befestigungstechnik von Strukturen auf harten Körperteilen,
- Figur 7: schematisch eine Ansicht einer trichterförmigen Landmarke mit aufgesetzter mit Mustern und magnetischen Materialien ausgestatteter Kalotte,
- Figur 8: eine schematische Anordnung aus einem weiteren mobilen Trackingsystem und einem medizinischen Instrument mit angebrachten Lokatoren, Strukturen, Mustern und einem plastisch deformierbaren Element,
- Figur 9: schematisch eine Ansicht eines Implantates mit plastisch deformierbaren Elementen und einem Muster,
- Figur 10: schematisch eine Anordnung aus einem mobilen Trackingsystem, einem Körperteil mit einer Struktur und Mustern und einem Spiegelsystem,
- Figur 11: schematisch eine Anordnung aus einem mobilen Trackingsystem, einem Scanner und einem Körperteil mit einer daran befestigten Struktur mit Mustern,
- Figur 12: schematisch eine Anordnung aus einem mobilen Trackingsystem, einem Projektor und einem Körperteil mit einer daran befestigten Struktur mit Mustern,
- Figur 13: schematisch eine Anordnung aus einem mobilen Trackingsystem, einem Ultraschallkopf und einem Körperteil mit einer daran angeordneten Struktur mit Mustern,
- Figur 14: schematisch eine Anordnung aus einem Bohr- und Fräskopf, einer Vorrichtung zur dreidimensionalen Erfassung von Knochen und Zähnen mittels Röntgenstrahlung und Strukturen an einer Schablone und/oder _Strukturen an Zähnen,
- Figur 15: schematisch eine Anordnung aus einem mobilen Trackingsystem und zwei mit Strukturen ausgestatteten Körperteilen.

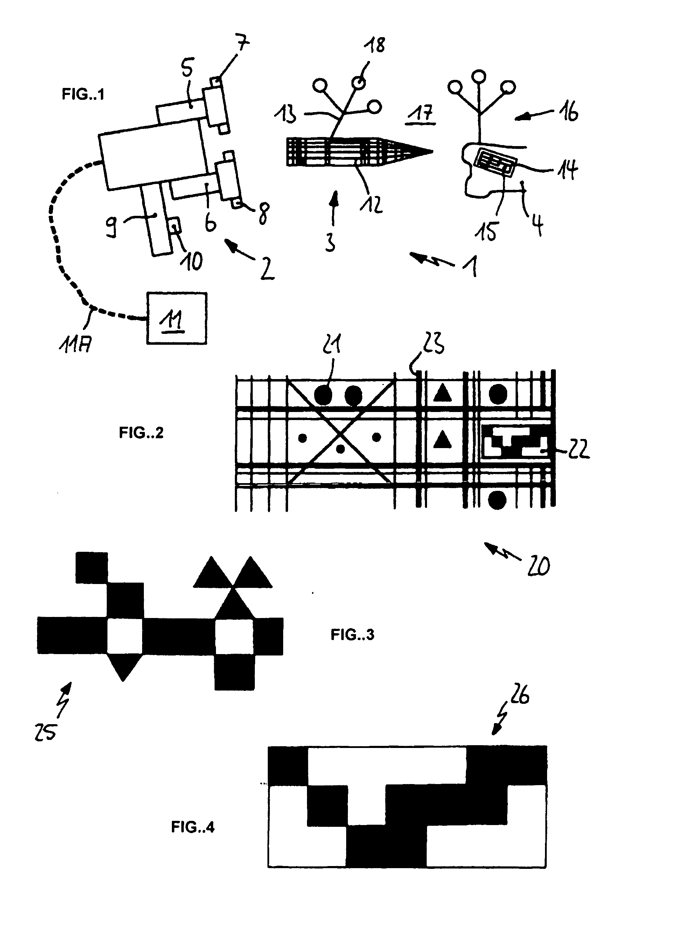

Die in der Figur 1 gezeigte Anordnung 1 umfasst ein mobiles Trackingsystem 2, eine medizinische Struktur 3 und einen Knochen 4 als menschliches Körperteil. Das mobile Trackingsystem 2 weist eine erste Kamera 5 und eine zweite Kamera 6 auf.

Um die medizinische Struktur 3 und den Knochen 4 gut ausleuchten zu können, weist das mobile Trackingsystem 2 darüber hinaus eine erste Beleuchtung 7 und eine zweite Beleuchtung 8 auf. Die Beleuchtungen 7 und 8 haben Leuchtdioden (hier nicht exemplarisch gezeigt), welche im infraroten Bereich strahlen. Alternativ können auch Leuchtdioden eingesetzt werden, welche beispielsweise im hyperroten Bereich strahlen.

Um das mobile Trackingsystem 2 gut halten und führen zu können, weist das Trackingsystem 2 einen Handgriff 9 auf, an welchem in diesem Ausführungsbeispiel eine Auslöseeinrichtung 10 vorgesehen ist. Mittels der Auslöseeinrichtung 10 kann auch die Messart gewählt werden. Vorliegend stehen drei Messarten zur Verfügung, nämlich eine Einzelmessung, eine Serie von Einzelmessungen und/oder eine Filmsequenz.

Dem mobilen Trackingsystem 2 ist zudem eine Auswerte- und Anzeigeeinheit 11 zugeordnet, die mittels einer Kommunikationsverbindung 11A mit dem mobilen Trackingsystem 2 in Verbindung steht. Von seiner Größe her ist das vorliegende mobile Trackingsystem 2 besonders leicht und somit handlich und gut tragbar ausgelegt.

Die Struktur 3 weist zum einen ein erstes Muster 12 und zum anderen einen ersten Lokator 13 auf. An dem Knochen 4 sind eine weitere Struktur 14 mit einem zweiten Muster 15 und ein Knochenlokator 16 befestigt. Die Strukturen 3 und 14 sind dreidimensionale Gebilde, auf denen das Muster 12 bzw. das Muster 15 aufgebracht ist. Die Muster 12 und 15 dienen dem mobilen Trackingsystem 2 zur Bestimmung der Raumlage der Strukturen 3 und 14. Insbesondere mittels des Musters 12 kann das mobile Trackingsystem 2 die Geometrie der Struktur 3 ermitteln.

Mittels des mobilen Trackingsystems 2 wird ein lokales Arbeitsgebiet 17 erfasst, welches die Struktur 3 und einen interessierenden Teilbereich des dargestellten Knochens 4 erfasst.

Über die Kommunikationsverbindung 11A wird das mobile Trackingsystem 2 mit Energie versorgt. Es versteht sich, dass anstelle der drahtgebundenen Kommunikationsverbindung 11A auch eine drahtlose Verbindung zwischen dem mobilen Trackingsystem 2 und der Auswerte- und Anzeigeeinheit 11 realisiert sein kann. In diesem Fall müsste das mobile Trackingsystem 2 eine eigene Energieversorgung umfassen und mit sich führen, welche beispielsweise mittels Batterien oder Brennstoffzellen realisiert sein könnte.

Sowohl der erste Lokator 13 als auch der Knochenlokator 16 sind in diesem Ausführungsbeispiel mit retro-reflektierenden Kugeln 18 ausgestattet. Die Form der ersten Struktur 3 und der weiteren Struktur 14 sowie die Eigenschaften des ersten Musters 12 und des zweiten Musters 15 sind bereits bei der Herstellung der Struktur 3 und der weiteren Struktur 14 definiert. Das mobile Trackingsystem 2 kann somit die Eigenschaften des ersten Musters 12 und des zweiten Musters 15 genau erkennen und eindeutig zuordnen.

Die Oberflächen der Struktur 3 und der weiteren Struktur 14 sind zumindest schmutzabweisend ausgebildet. Hierdurch werden Messresultate des mobilen Trackingsystems 2 weniger durch Verschmutzungen, wie beispielsweise Blutspritzer oder verunreinigte Luft, beeinträchtigt. Eine möglicherweise erforderliche Reinigung der Oberflächen wird durch die schmutzabweisenden Oberflächen zudem wesentlich vereinfacht.

Das in der Figur 2 gezeigte Muster 20 weist für das mobile Trackingsystem 2 eine Vielzahl an Linien unterschiedlicher Breite, Kreise, Ellipsen, Dreiecke und Rechtecke auf. Einige Teilbereiche 21 des Musters 20 dienen vorliegend dazu, die Algorithmuseffizienz des mobilen Trackingsystems 2 zu steigern. Ein optional vorhandener Teilbereich 22 des Musters 20 dient zur Identifikation einer dem Muster 20 zugeordneten Struktur. In dem Teilbereich 22 ist insbesondere die Relation einer Struktur zu einem medizinischen Instrument oder zu einem medizinischen Werkzeug definiert.

Darüber hinaus umfasst das Muster 20 eine Vielzahl an Linien 23 (hier nur exemplarisch beziffert) mit unterschiedlicher Breite. Die Linien 23 haben vorteilhafter Weise eine ausreichend dimensionierte Breite, wie vorliegend beispielsweise vier bis zehn Pixel eines verwendeten Kamerasensors, so dass ein Sensorbild des Musters 20 und damit auch Strukturgeometrien ausreichend genau bestimmt werden können.

Vorteilhafter Weise umfasst das Muster 20 breite und dünnere Linien 23, so dass das Muster 20 immer gut zu erkennen ist. Sind beispielsweise Fokuslängen von Kameras fix vorgegeben und variiert der Abstand zwischen einem Körper und einem Trackingsystem während einer Arbeitsphase stark, ist es vorteilhaft, wenn das Muster 20 bevorzugt aus Linien 23 verschiedener Breite besteht. Die breiteren Linien des Musters 20 werden vor allem bei großen Distanzen zwischen einem Körper und einem mobilen Trackingsystem verwendet. Die dünneren Linien des Musters 20 werden hingegen vor allem bei kleineren Distanzen zwischen einem Körper und einem mobilen Trackingsystem verwendet.

Die in den Figuren 3 und 4 dargestellten weiteren Muster 25 und 26 zeigen die mögliche Vielfalt an Mustern, die vorliegend zur Anwendung kommen können.

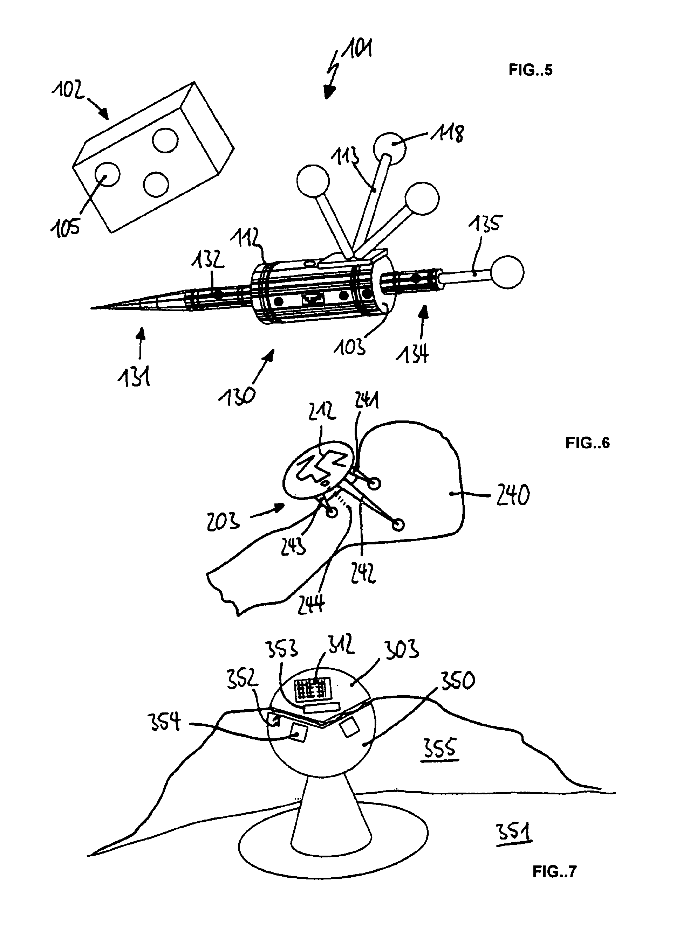

Die Anordnung 101 nach Figur 5 zeigt ein mobiles Trackingsystem 102 in unmittelbarer Umgebung eines medizinischen Instrumentes 130.

Das mobile Trackingsystem 102 weist drei Kameras 105 (hier nur exemplarisch beziffert) auf.

Das medizinische Instrument 130 ist als Einsatz zum Anordnen in einer Struktur 103 ausgebildet. Der Einsatz ist vorliegend in die Struktur 103 eingeschoben. Das medizinische Instrument 130 hat eine Instrumentenspitze 131. Das medizinische Instrument 130 mitsamt der Instrumentenspitze 131 weist ein Instrumentenmuster 132 auf. Die Instrumentenstruktur 103 hat ein Instrumentenstrukturmuster 112.

An der Instrumentenstruktur 103 ist ein Instrumentenstrukturlokator 113 befestigt. Der Instrumentenstrukturlokator 113 umfasst neben retro-reflektierenden Kugeln 118 einen Befestigungsfuß 133, mittels welchem der Instrumentenstrukturlokator 113 an der Instrumentenstruktur 103 befestigt ist.

Das medizinische Instrument 130 weist an seinem der Instrumentenspitze 131 gegenüberliegenden Instrumentenende 134 einen Instrumentenlokator 135 auf.

Das hier in der Figur 5 gezeigte medizinische Instrument 130 ist ein Beispiel für einen Körper in Form eines medizinischen Instrumentes 130, welches mit einer Vielzahl an Mustern 112, 132 und Lokatoren 113, 135 ausgestattet ist.

Das mobile Trackingsystem 102 kann anhand der unterschiedlichen Muster 112, 132 eindeutig die Geometrien des medizinischen Instrumentes 130 bestimmen. Darüber hinaus bestimmt das mobile Trackingsystem 102 die Lokatorpositionen bezüglich der Muster 112, 132 an dem medizinischen Instrument 130. Dies hat den Vorteil, dass mit der Vermessung der Lokatoren 113, 135 auch beispielsweise die Position der Instrumentenspitze 131 bekannt ist.

An dem in der Figur 6 gezeigten Knochenteil 240 ist eine Struktur 203 mit einem Muster 212 befestigt. Die Struktur 203 umfasst in diesem Ausführungsbeispiel eine erste Stütze 241, eine zweite Stütze 242 und eine dritte Stütze 243.

Zusätzlich verfügt die Struktur 240 über eine Schraube 244, so dass die Struktur 203 an dem Knochenteil 240 geschraubt und besonders stabil an dem Knochenteil 240 befestigt ist. Vorliegend ist die Struktur 203 als zweidimensionale Platte ausgebildet, auf der das Muster 212 aufgebracht ist.

Die in der Figur 7 gezeigte Landmarke 350 ist auf eine Haut 351 eines Patienten aufgeklebt. Die Landmarke 350 hat eine trichterförmige Einbuchtung 352. In die trichterförmige Einbuchtung 352 der Landmarke 350 ist eine Struktur 303 eingesetzt. Die Struktur 303 weist ein Muster 312 auf. Die Befestigung der Struktur 303 an der Landmarke 350 erfolgt mittels eines Magneten 353. Der Magnet 353 der Struktur 302 wirkt mit einem ferromagnetischen Material 354 der Landmarke 350 zusammen. Zwischen der Landmarke 350 und der Struktur 302 ist ein Operationstuch 355 eingeklemmt.

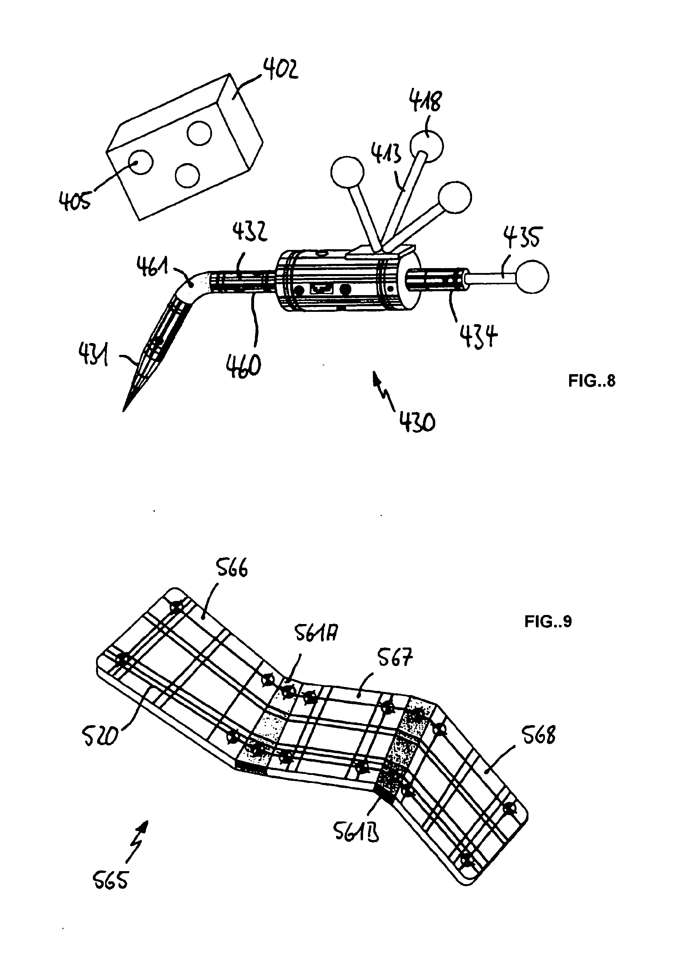

Das in der Figur 8 gezeigte medizinische Instrument 430 umfasst einen Instrumenteneinsatz 460, der zwischen einer Instrumentenspitze 431 und einem Instrumentenende 434 ein plastisch verformbares Element 461 aufweist. Bis auf das plastisch verformbare Element 461 ist der komplette Instrumenteneinsatz 460 mit einem Instrumentenmuster 432 versehen.

Es versteht sich, dass in einem weiteren Ausführungsbeispiel auch nur einzelne Teilbereiche mit derartigen Mustern versehen sein können.

Am Instrumentenende 434 ist ein Instrumentenlokator 435 vorgesehen. Darüber hinaus ist ein Instrumentenstrukturlokator 413 am medizinischen Instrument 430 befestigt. Sowohl der Instrumentenlokator 435 als auch der Instrumentenstrukturlokator 413 verfügen über retro-reflektierende Kugeln 418 (hier nur exemplarisch beziffert).

In unmittelbarer Nähe des medizinischen Instruments 430 befindet sich ein mobiles Trackingsystem 402 mit drei Kameras 405 (hier nur explizit beziffert), mittels welchem alle an dem medizinischen Instrument 430 befindlichen Muster 432 und Lokatoren 413, 435 ermittelt werden. So kann beispielsweise auch eine Verlagerung der Instrumentenspitze 431 gegenüber weiteren Bereichen des medizinischen Instrumentes 430 erfasst und bestimmt werden.

Das in der Figur 9 gezeigte Implantat 565 umfasst ein erstes verformbares Element 561A und ein zweites verformbares Element 561 B. Das Implantat 565 ist hierdurch in einen ersten Teilbereich 566, einem zweiten Teilbereich 567 und einem dritten Teilbereich 568 unterteilt. Die einzelnen Teilbereiche 566, 567 und 568 sind somit zueinander relativ verlagerbar. Um die komplette Implantatgeometrie des vorliegenden Implantats 565 zu erfassen und zu bestimmen, ist das gesamte Implantat 565 mit einem Muster 520 überzogen.

Das Implantat 565 kann vorliegend nun solange verformt und von einem mobilen Trackingsystem ausgemessen werden, bis es letztendlich eine gewünschte Form erreicht hat. Das Implantat 565 kann dann mittels Befestigungsschrauben (hier nicht gezeigt) an einem weiteren Körperteil (hier nicht gezeigt) befestigt werden.

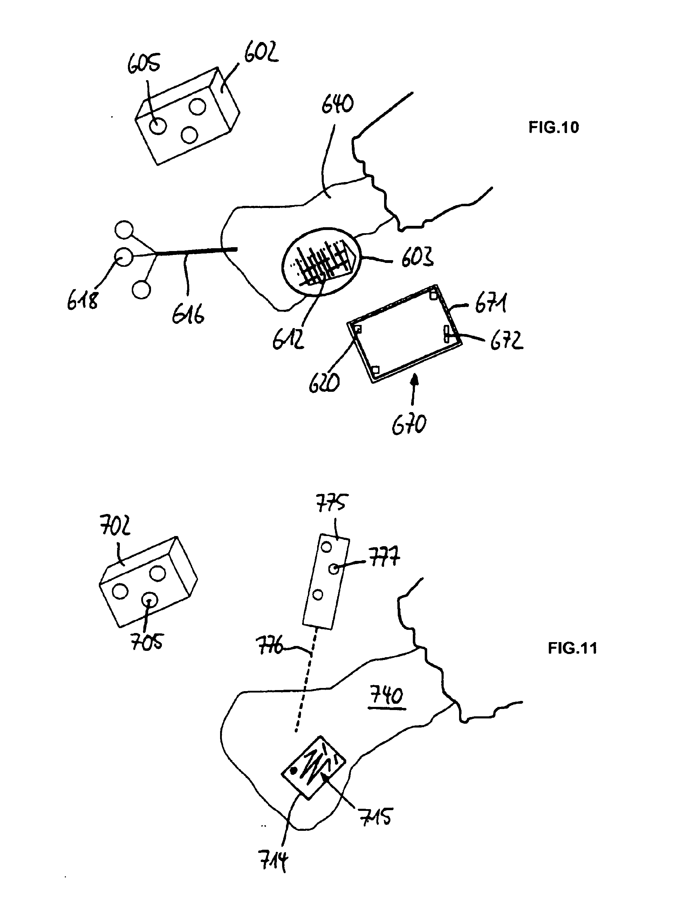

An dem in der Figur 10 gezeigten Knochenteil 640 ist eine Struktur 603 mit einem darauf angeordneten Muster 612 sowie ein Knochenlokator 616 mit retro-reflektierenden Kugeln 618 befestigt. Das Knochenteil 640, die Struktur 603 und der Knochenlokator 616 werden mittels eines mobilen Trackingsystems 602, welches mit Kameras 605 (hier nur exemplarisch beziffert) ausgestattet ist, vermessen.

Da die Struktur 603 mit dem Muster 612 von dem Knochenteil 640 zumindest teilweise überdeckt ist, so dass insbesondere das Muster 612 von dem mobilen Trackingsystem 602 nicht unmittelbar erfasst werden kann, ist gegenüber dem Muster 612 ein Spiegel 670 angeordnet. Somit kann das mobile Trackingsystem 602 mittels des Spiegels 670 das Muster 612 auf der Struktur 603 sehen und erfassen.

Der Spiegel 670 umfasst zu seiner Identifizierung zum einen einen markierten Randbereich 671 sowie zum anderen einen Identifikator 672 und weitere Muster 620 (hier nur exemplarisch beziffert).

An dem in der Figur 11 gezeigten Knochenteil 740 ist eine Knochenstruktur 714 mit einem Muster 715 befestigt. In unmittelbarer Nähe des Knochenteils 740 befindet sich ein mobiles Trackingsystem 702 mit drei Kameras 705 (hier nur exemplarisch beziffert).

Darüber hinaus befindet sich in der unmittelbaren Nähe des Knochenteils 740 auch ein handgeführter optischer Scanner 775, welcher die Oberflächentopologie des Knochenteils 740 mit einem Lichtstrahl 776 vermisst. Die Orientierung und die Lage des optischen Scanners 775 und der Knochenstruktur 714 wird mittels des Trackingsystems 702 gemessen. Hierzu weist der optische Scanner 775 drei aktive LEDs 777 (hier nur exemplarisch beziffert) auf. Die vom optischen Scanner 765 vermessene Oberflächentopologie des Knochenteils 740 wird zusätzlich mittels bereits vorliegenden CT- oder MRI-Bilder referenziert.

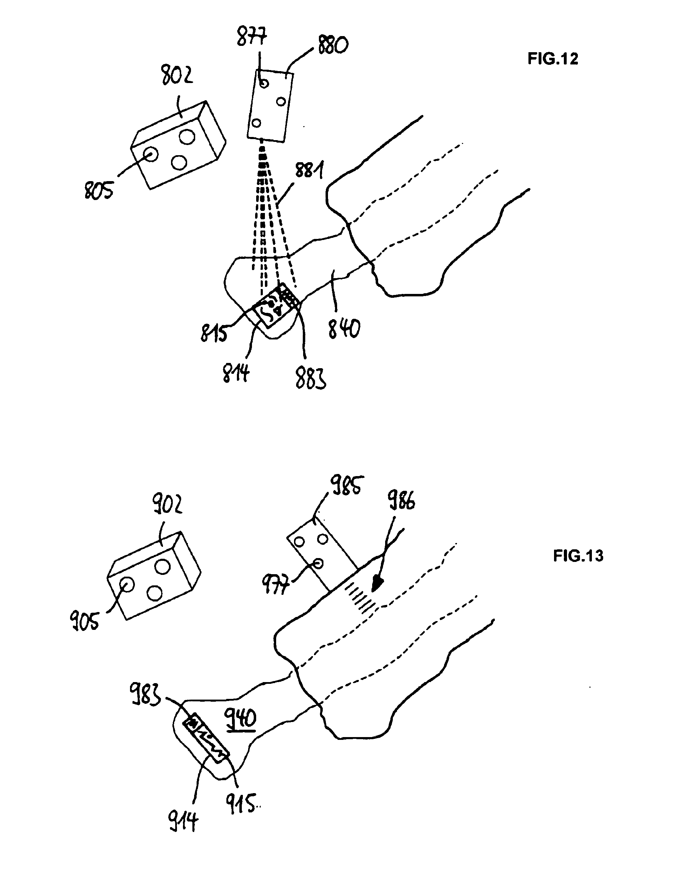

An dem in der Figur 12 gezeigten Knochenteil 840 ist eine Knochenstruktur 814 mit einem Muster 815 und mit einem sekundären Muster 883 befestigt. Das sekundäre Muster 883 kann in diesem Ausführungsbeispiel von einem CT (hier nicht dargestellt) erfasst werden, so dass die Raumlage des sekundären Musters 883 bezüglich des Knochenteils 840 auf Grund der mit dem CT gewonnenen Daten bekannt ist. Vor dem Knochenteil 840 ist ein mobiles Trackingsystem 802 mit drei Kameras 805 (hier nur exemplarisch beziffert) gehalten.

Ebenfalls in unmittelbarer Nähe des Knochenteils 840 wird ein Projektor 880 gehalten, der mehrere räumlich definierte Lichtstrahlen 881 (hier nur exemplarisch beziffert) auf das Knochenteil 840 projiziert. Der Projektor 880 umfasst drei aktive LEDs 877 (hier nur exemplarisch beziffert), um von dem mobilen Trackingsystem 802 hinsichtlich seiner Raumlage genau erfasst werden zu können. Die mittels der Lichtstrahlen 881 zusätzlich beleuchteten Stellen des Knochenteils 840 werden vom mobilen Trackingsystem 802 gemessen und als Raumpositionen berechnet. Die daraus ermittelte Oberflächentopologie des Knochenteils 840 kann mit CT- oder MRI-Bildern referenziert werden.

An dem in Figur 13 gezeigten Knochenteil 940 ist eine Knochenstruktur 914 mit einem Muster 915 und mit einem sekundären Muster 983 befestigt. In unmittelbarer Nähe des Knochenteils 940 ist ein mobiles Trackingsystem 902 mit drei Kameras 905 (hier nur exemplarisch beziffert) gehalten. Mittels des mobilen Trackingsystems 902 sind das Muster 915 und das sekundäre Muster 983 ermittelbar. Das sekundäre Muster 983 kann darüber hinaus noch von weiteren bildgebenden Geräten (hier nicht gezeigt), wie etwa ein CT- oder MRI-Gerät, erfasst werden. An dem Knochenteil 940 ist ein Ultraschallmesskopf 985 geführt, welcher mittels einer Schallkeule 986 das Knochenteil 940 vermisst. Der Ultraschallmesskopf 985 umfasst hierbei drei aktive LEDs 977 (hier nur exemplarisch beziffert), um von dem mobilen Trackingsystem 902 erfasst zu werden. Die von dem Ultraschallmesskopf 985 gemessene Knochenoberfläche und der Knochenaufbau werden vorliegend bezüglich CT- oder MRI-Bildern referenziert.

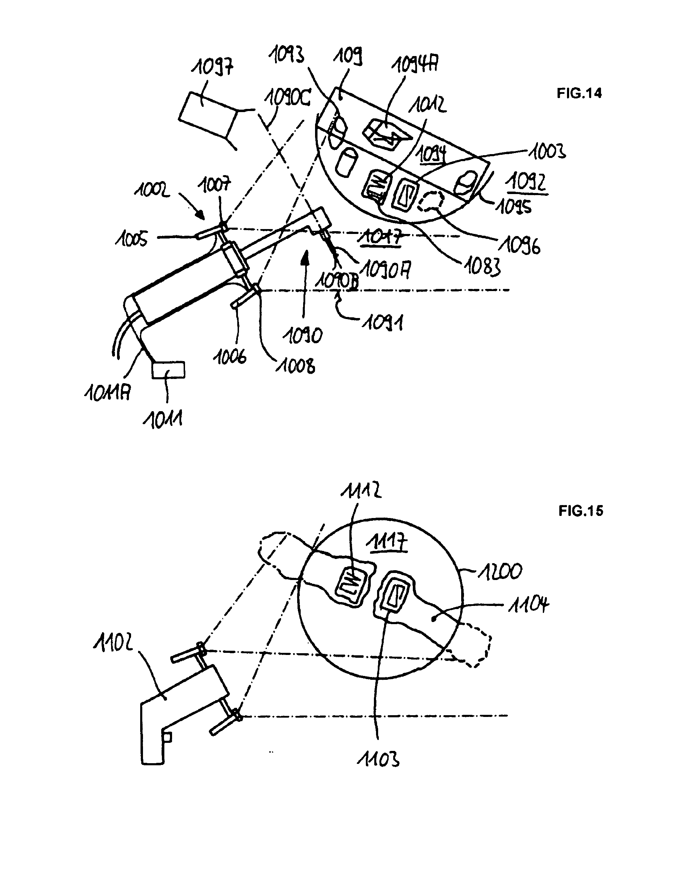

Das in der Figur 14 gezeigte mobile Trackingsystem 1002 ist auf einem medizinischen Werkzeug 1090, welches vorliegend ein Bohrgerät mit einem Bohreinsatz 1090A ist, fixiert. Besonders vorteilhaft ist es in diesem Ausführungsbeispiel, dass das mobile Trackingsystem 1002 eine besonders kleine und leichte Ausführung ist, so dass es problemlos auf dem Werkzeug 1090 fixiert werden kann.

Das mobile Trackingsystem 1002 umfasst eine erste leichte, kleine Kamera 1005 und eine zweite leichte, kleine Kamera 1006, welche jeweils einen optischen Öffnungswinkel 1091 (hier nur beispielhaft beziffert) aufweist.

Ermittelte Messdaten werden über eine Kommunikationsverbindung 1011A an eine Auswerte- und Anzeigeeinheit 1011 geleitet. Die Kommunikationsverbindung 1011A beinhaltet in diesem Ausführungsbeispiel auch die Energieversorgung (hier nicht gezeigt) des Werkzeuges 1090 und eine Wasserzufuhr (hier nicht gezeigt) zum Freispülen eines lokalen Arbeitsgebietes 1017 am Werkzeug 1090.

Eine alternative Ausführungsvariante kann vorsehen, dass die Optiken (hier nicht explizit dargestellt) der Kameras 1005, 1006 unmittelbar am Werkzeug 1090 angebracht und die restlichen Komponenten der Kameras 1005, 1006, wie etwa ein Flächensensor (hier nicht dargestellt), in der Auswerte- und Anzeigeeinheit 1011 untergebracht sind. Mittels der Kommunikationsverbindung 1011A, welche vorliegend aus einem Lichtfaserbündel besteht, sind die Kameras 1005, 1006 mit den Flächensensoren optisch verbunden.

Das vorliegende mobile Trackingsystem 1002 sieht einen Kieferbereich 1092 während eines Eingriffs ein. Sowohl eine erste Beleuchtung 1007 als auch eine zweite Beleuchtung 1008 sorgen vorliegend für eine ausreichende Ausleuchtung von Strukturen 1003 (hier nur exemplarisch beziffert) mit darauf angeordneten Mustern 1012 (hier nur exemplarisch beziffert) sowie mit darauf angeordneten sekundären Mustern 1083 (hier nur exemplarisch beziffert), des Weiteren von Zähnen 1093 (hier nur exemplarisch beziffert), welche im Kieferbereich 1092 angeordnet sind.

Die Strukturen 1003 sind in diesem Ausführungsbeispiel auf geeignete Zähne 1093 fixiert. Somit ist ein sicherer Halt der Strukturen 1003 an den Zähnen 1093 gewährleistet.

Das mobile Trackingsystem 1002 orientiert sich anhand der temporär auf einigen geeigneten Zähnen 1093 angebrachten Strukturen 1003, welche entsprechende Muster 1012 aufweisen.

Besonders vorteilhaft ist es, dass auf Grund der verwendeten Strukturen 1003 mit den darauf angebrachten Mustern 1012 und sekundären Mustern 1083 eine Verwendung von Lokatoren nicht weiter erforderlich ist.

Alternativ hierzu kann eine vor dem Eingriff hergestellte Schablone 1094 mit darauf angebrachten Schablonenmustern 1094A verwendet werden. Die Schablone 1094 ist dann an einzelnen Zähnen 1093 eines Unterkiefers 1095 oder eines Oberkiefers (hier nicht gezeigt) fixiert. Die Schablone 1094 weist vorliegend eine Ansetzposition 1096 für das medizinische Werkzeug 1090 auf.

Gegebenenfalls müssen die Strukturen 1003 direkt im Kieferknochen 1095 (hier nur exemplarisch der Unterkieferknochen gezeigt) verankert werden. Wenn die Geometrie des Werkzeuges 1090 mit dem optionalen Einsatz 1090A bezüglich des mobilen Trackingsystems 1002 kalibriert werden muss, wird das Werkzeug 1090 mit einer Kalibriervorrichtung (hier nicht dargestellt) mit angebrachten Strukturen 1003 in definierter Art und Weise mechanisch verbunden. Das mobile Trackingsystem 1002 bestimmt dann anhand dieser Strukturen 1003 seine relative Raumlage zur Kalibriervorrichtung und daraus die Geometrie des Werkzeuges 1090 mit dem optionalen Einsatz 1090A. Damit ist beispielsweise die Position einer Bohrerspitze 1090B und die Orientierung der Bohrerachse 1090C bezüglich des mobilen Trackingsystems 1002 bekannt.

Insbesondere beim Setzen von Zahnimplantaten (hier nicht explizit gezeigt) wird die Ansetzposition 1096 des Bohrers 1090A, die Bohrachse 1090C und eine Bohrtiefe vorteilhafter Weise aus weiteren Patientendaten gewonnen. Ein entsprechendes Verfahren wird im Folgenden beispielhaft näher erläutert.

Die für einen Patienten optimale Konfiguration eines Implantats und einer aufgesetzten Krone, wie etwa die Form, die Farbe, die Position oder die Krafteinlenkung eines belasteten Ersatzzahns, auf das Implantat im Knochen basieren im Wesentlichen auf der Oberfläche und der Volumenstruktur der bestehenden Zahnreihen, des Kiefers und dem Weichgewebe. Diese Details werden mittels optischen Abtastens (Scannen) und mit der dreidimensionalen Erfassung der entsprechenden Körperteile mittels Röntgenstrahlung gewonnen.

Nach dem optischen Abtasten der entsprechenden Körperteile werden die Muster 1012 und die hier vorgesehenen röntgen-opake Sekundärmuster 1083 aufweisenden Strukturen 1003 an einigen Zähnen 1093 fixiert. Diese Strukturen 1003 können Schablonen 1094 oder direkt an einzelnen geeigneten Zähnen 1093 aufgebrachte Strukturen 1003 sein. Die Muster 1012 und Sekundärmuster 1083 können auch direkt auf Zähnen 1093 aufgedruckt werden.

Mittels Röntgenstrahlung werden sowohl dreidimensionale Strukturen von Knochen 1095, Zähnen 1093 oder der Verlauf von Nervenbahnen als auch die Sekundärmuster 1083 erfasst. Durch eine Registrierung werden die Datensätze aus optischem Abtasten (Oberflächen) und der Röntgenstrahlmessung (Kiefer und Zahnstruktur) in ein gemeinsames Koordinatensystem überführt. Damit entsteht ein Modell des betrachteten Körperteils, in welchem alle erforderlichen Details, wie die Knochenstruktur 1095, die Zähne 1093, die Nervenbahnen, Weichteiloberflächen, die Muster 1012 und die Sekundärmuster 1083, enthalten sind. Insbesondere sind einem Koordinatensystem des mobilen Trackingsystems durch Erfassen der Muster 1012 auch die Lage der Kieferknochen 1095, der Zähne 1093 und der Weichteiloberflächen bekannt.

Mit Hilfe dieses Modells wird mittels geeigneter Design-Software die optimale Konfiguration des Implantats und der Krone sowie die Ansetzposition 1096 des Bohrers 1090A, die Bohrachse 1090C und die Bohrtiefe ermittelt.

Das Implantat liegt idealerweise vorgefertigt am Lager. Die Krone wird anschliessend mit geeigneten Mitteln vorteilhafter Weise im gleichen Operationsraum aus Halbfabrikaten CAD/CAM unterstützt gefertigt. Hierbei muss kein nach dem heutigen Stand der Technik benötigter Abdruck für die Kronenherstellung erzeugt werden.

Während der Fertigstellung der Krone wird der eigentliche Haupteingriff am Patienten durchgeführt. Hierbei ermittelt eine übergeordnete Software für die Navigation mit dem mobilen Trackingsystem 1002 die Ansetzposition 1096 des Bohrers 1090A, die Bohrachse 1090C und die Bohrtiefe im Koordinatensystem des mobilen Trackingsystems 1002. Das mobile Trackingsystem 1002 liefert permanent die Raumlage beispielsweise des Bohrers 1090A relativ zur Kieferanatomie. Diese Informationen können beispielsweise mittels akustischer und/oder optischer Signale zur Verfügung gestellt werden. Der Chirurg kann diese Information während des Bohrvorgangs dauernd verwenden. Er kann sie alternativ auch nur während einer kritischen Phase, wie beispielsweise zu Beginn und/oder in der Endphase des Eingriffes verwenden.

Das Implantat kann unmittelbar nach dem Bohren in das vorbereitete Loch geschraubt werden. Anschliessend wird die zwischenzeitlich fertig gestellte Krone auf oder an das Implantat fixiert und die Operationsstelle weiter versorgt.

Das Verfahren wird vorteilhafter Weise so gestaltet, dass das optische Abtasten, die Fixierung der Strukturen 1003 mit den Mustern 1012, 1083, das dreidimensionale Erfassen von Knochen 1095 und Zähnen 1093 mittels Röntgenstrahlung, die Erstellung des Modells des Körperteils, der optimalen Konfiguration des Implantats und der Krone, die Herstellung der Krone, das mit Hilfe des mobilen Trackingsystems 1003 kontrolliert gebohrte Loch im Kiefer 1095, das Setzen des Implantates und die Fixierung der Krone in einer Sitzung erfolgen kann.

Insbesondere bei kieferchirurgischen Eingriffen, wie beim Setzen vom Implantaten, muss darauf geachtet werden, dass beispielsweise Nerven nicht beschädigt werden. Deshalb haben sich Hilfsmittel, wie Navigationssysteme auf Basis von Trackingsystemen, bewährt. Diese helfen einem Operateur beim Eingriff sehr gut zielgerichtet zu arbeiten. Das hier näher beschriebene Verfahren kommt vorteilhafter Weise zudem ohne retro-reflektierende Kugeln aus.

Das in der Figur 15 gezeigte mobile Trackingsystem 1102 wird bevorzugt bei minimalinvasiven Operationstechniken eingesetzt. Bei minimalinvasiven Operationstechniken wird das lokale Operationsgebiet 1117 mit einer kleinen Öffnung 1200 freigelegt. Das heißt, dass das eigentliche lokale Arbeitsgebiet 1117 für das mobile Trackingsystem 1102 relativ klein ist, was den Einsatz von geometrisch kleinen Trackingsystemen sinnvoll macht. Der Chirurg setzt das mobile Trackingsystem 1102 lokal und nach Bedarf ein. Das bedeutet, vor und nach bestimmten Arbeitsschritten wird das mobile Trackingsystem 1102 eingesetzt.. Dies ist vorteilhaft, da es bei Nichtgebrauch aus dem Operationsgebiet 1117 weggelegt wird und somit im unmittelbaren Operationsumfeld keinen Platz einnimmt. Bei Bedarf kann das mobile Trackingsystem 1102 auch in kritischen Phase während eines Arbeitsschrittes eingesetzt werden.

Ein typischer Ablauf einer minimalinvasiven Operation, bei der eine Registrierung nicht benötigt wird, kann wie folgt ablaufen. Es wird die kleine Öffnung 1200 freigelegt. Anschließend werden Strukturen 1103 (hier nur exemplarisch beziffert) mit ihren Mustern 1112 in geeigneter Weise an Gelenkknochen 1104 befestigt. Nun wird die relative Raumlage der Strukturen 1103 hinsichtlich einer Ausgangslage vermessen. Dann erfolgt der eigentliche chirurgische Eingriff mit einer eventuellen Montage von Hilfsimplantaten (hier nicht gezeigt). Des Weiteren wird das definitive Implantat (hier nicht gezeigt) eingesetzt und eine erneute Vermessung und Beurteilung der relativen Raumlage der Strukturen 1103 vorgenommen. Letztendlich werden alle Strukturen 1103 entfernt.

Das hier beschriebene Trackingsystem 1102 ist ergonomisch sehr gut ausgebildet. Zudem ist es kostengünstig bereitgestellt. Mittels der hier verwendeten Strukturen ist eine weitere Verwendung von meist recht sperrig bauenden Lokatoren vorteilhafter Weise nicht erforderlich.

Bei allen vorbeschriebenen Verfahren können Instrumente, Werkzeuge und/oder Hilfsmittel, wie sie insbesondere auch in den vorstehenden Figuren erläutert sind, zum Einsatz gelangen.

An dieser Stelle sei nochmals angemerkt, dass die beschriebene Vorrichtung und das beschriebene Verfahren keineswegs auf die Medizintechnik beschränkt sind. Insbesondere ist das vorliegende Verfahren für die Mess- und Fertigungstechnik sowie für die Handhabung oder Bearbeitung von Objekten und in der Qualitätskontrolle vorteilhaft geeignet

## Patentansprüche

1. Vorrichtung zum berührungslosen Ermitteln und Vermessen einer Raumposition und/oder einer Raumorientierung von Körpern, mit Körpern (3, 4), wie einem medizinischen Werkzeug, einem Werkstück und/oder einem medizinischen Instrument, und mit einem Trackingsystem, mittels welchem die Körper lokalisiert und zueinander in Relation gebracht werden, wobei das Trackingsystem oder zumindest Bauteile oder Bauteilgruppen davon mobil einsetzbar sind, wobei die Vorrichtung Mittel zum Berechnen einer Verlagerung des mobilen Trackingsystems (2) gegenüber den Körpern (3, 4) aufweist und das Trackingsystem mindestens zwei Kameras (5, 6), eine Optik und eine Sensorik aufweist, und wobei an den Körpern (3, 4) Muster (12, 15) in Form von im Wesentlichen zweidimensionalen Gebilden angeordnet sind, die vom mobilen Trackingsystem ermittelt werden.

2. Vorrichtung nach Anspruch 1, ***dadurch gekennzeichnet, dass*** die Muster aus einer Kombination aus reflektierenden, absorbierenden, fluoreszierenden oder selbstleuchtenden Materialien bestehen.

3. Vorrichtung nach einem der vorstehenden Ansprüche, ***dadurch gekennzeichnet, dass*** die Muster eine auf eine Struktur aufgebrachte Farbschicht sind.

4. Vorrichtung nach einem der vorstehenden Ansprüche, ***dadurch gekennzeichnet, dass*** das mobile Trackingsystem (2) ein Gewicht von weniger als 0,1 kg, aufweist.

5. Vorrichtung nach einem der vorstehenden Ansprüche, ***dadurch gekennzeichnet, dass*** das mobile Trackingsystem (2) eine Befestigungseinrichtung zum Anordnen an einer verlagerbaren Führungseinrichtung, wie einem manuell verlagerbaren Haltearm und/oder einem Roboterarm, aufweist.

6. Vorrichtung nach einem der vorstehenden Ansprüche, ***dadurch gekennzeichnet, dass*** das mobile Trackingsystem (2) einen Handgriff (9) aufweist.

7. Vorrichtung nach einem der vorstehenden Ansprüche, ***dadurch gekennzeichnet, dass*** die Berechnungsmittel als Software ausgeführt sind.

8. Vorrichtung nach einem der vorstehenden Ansprüche, ***dadurch gekennzeichnet, dass*** das mobile Trackingsystem (2) eine eigene Energiequelle aufweist.

9. Vorrichtung nach einem der vorstehenden Ansprüche, ***dadurch gekennzeichnet, dass*** das mobile Trackingsystem (2) Mittel zur drahtlosen Kommunikation aufweist.

10. Vorrichtung nach einem der vorstehenden Ansprüche, ***dadurch gekennzeichnet, dass*** das mobile Trackingsystem (2) Mittel zum Beleuchten (7, 8) aufweist.

11. Vorrichtung nach einem der vorstehenden Ansprüche, ***dadurch gekennzeichnet, dass*** das mobile Trackingsystem (2) auf einem Werkzeug oder einem medizinischen Instrument angeordnet ist.

12. Verfahren zum berührungslosen Ermitteln und Vermessen einer Raumposition und/oder einer Raumorientierung von Körpern mit einer Vorrichtung nach einem der vorhergehenden Ansprüche, ***dadurch gekennzeichnet, dass*** das mobile Trackingsystem während seiner Verwendung manuell oder an einem Roborterarn montiert automatisiert gegenüber Körpern bewegt und verlagert wird.

13. Verfahren nach Anspruch 12, ***dadurch gekennzeichnet, dass*** zusammen mit dem mobilen Trackingsystem (2) ein mobiler optischer Scanner oder ein mobiler Projektor für strukturiertes Licht verwendet wird.

14. Verfahren nach einem der Ansprüche 12 oder 13, ***dadurch gekennzeichnet, dass*** das mobile Trackingsystem (2) während seiner Verwendung, insbesondere händisch, unmittelbar vor die zu erfassenden lokalen Arbeitsgebiete gehalten wird.

15. Verfahren nach einem der Ansprüche 12 bis 14, ***dadurch gekennzeichnet, dass*** zumindest die Raumposition und/oder die Raumorientierung zwischen wenigstens zwei Körpern (4, 13, 14, 16) lediglich phasenweise ermittelt wird.

16. Verfahren nach einem der Ansprüche 12 bis 15, ***dadurch gekennzeichnet, dass*** eine Geometrie eines Körpers (4, 13, 14, 16) ermittelt und vermessen wird.

17. Verfahren nach einem der Ansprüche 12 bis 15, ***dadurch gekennzeichnet, dass*** die Körper (3, 4,13, 14, 16) mittels auf und/oder an den Körpern (3, 4, 13, 14, 16) befindlichen Mustern (12, 15) und/oder sekundären Mustern, welche von weiteren bildgebenden Systemen erkannt werden, registriert werden.

18. Verfahren nach einem der Ansprüche 12 bis 16, ***dadurch gekennzeichnet, dass*** zumindest die Raumposition und/oder die Raumorientierung zwischen wenigstens zwei Körpern (3, 4, 13, 14, 16) in einer ersten Messung ermittelt wird, wenigstens ein Körper (3,4,13,14,16) bearbeitet wird und zumindest die Raumposition und/ oder die Raumorientierung zwischen wenigstens zwei Körpern (3,4,13,14,16) in einer weiteren Messung ermittelt wird.

19. Verfahren nach einem der Ansprüche 12 bis 17, ***dadurch gekennzeichnet, dass*** ein Körper ein Zahn ein anderes knöchernes Körperteil, ein Implantat oder eine Schablone ist.

## Claims

1. A device for contactless determination and measurement of a spatial position and/or spatial orientation of bodies, having bodies (3, 4) such as a medical tool, a workpiece and/or a medical instrument, and having a tracking system by means of which the bodies are located and correlated with each other, wherein the tracking system, or at least components or component groups thereof are capable of mobile deployment, wherein the device includes means for calculating a displacement of the mobile tracking system (2) relative to the bodies (3, 4), and the tracking includes at least two cameras (5, 6) an optical system and a sensor system, and wherein patterns (12, 15) in the form of substantially two-dimensional constructs are arranged on the bodies (3, 4) and are identified by the mobile tracking system.

2. The device according to claim 1, ***characterized in that*** the patterns consist of a combination of reflective, absorbent, fluorescing or other self-luminous materials.

3. The device according to either of the preceding claims, ***characterized in that*** the models are a colour layer applied to a structure.

4. The device according to any one of the preceding claims, ***characterized in that*** the mobile tracking system (2) weighs less than 0.1 kg.

5. The device according to any one of the preceding claims, ***characterized in that*** the mobile tracking system (2) has a fastening apparatus so that it can be attached to a movable guidance device, such as a manually movable support arm and/or a robot arm.

6. The device according to any one of the preceding claims, ***characterized in that*** the mobile tracking system (2) has a handle (9).

7. The device according to any one of the preceding claims, ***characterized in that*** the calculation means are in the form of computer software.

8. The device according to any one of the preceding claims, ***characterized in that*** the mobile tracking system (2) has its own power source.

9. The device according to any one of the preceding claims, ***characterized in that*** the mobile tracking system (2) includes means for wireless communication.

10. The device according to any one of the preceding claims, ***characterized in that*** the mobile tracking system (2) includes lighting means (7, 8).

11. The device according to any one of the preceding claims, ***characterized in that*** the mobile tracking system (2) is mounted on a tool or a medical instrument.

12. A method for contactless determination and measurement of a spatial position and/or spatial orientation of bodies with a device according to any one of the preceding claims, ***characterized in that*** when in use the mobile tracking system is movable and displaceable relative to bodies manually or automatically by mounting on a robot arm.

13. The method according to claim 12, ***characterized in that*** a mobile optical scanner or a mobile projector is used for structured light in conjunction with the mobile tracking system (2).

14. The method according to either of claims 12 or 13, ***characterized in that*** when in use the mobile tracking system (2) is held, particularly by hand, immediately in front of the local work sites to be scanned.

15. The method according to any one of claims 12 to 14, ***characterized in that*** at least the spatial position and/or the spatial orientation between at least two bodies (4, 13, 14, 16) only in phases.

16. The method according to any one of claims 12 to 15, ***characterized in that*** a geometry of a body (4, 13, 14, 16) is determined and measured.

17. The method according to any one of claims 12 to 15, ***characterized in that*** the bodies (3, 4, 13, 14, 16) are registered by means of patterns (12, 15) located on and/or attached to the bodies (3, 4, 13, 14, 16) and/or by secondary patterns that are detected by other imaging systems.

18. The method according to any one of claims 12 to 16, ***characterized in that*** at least the spatial position and/or spatial orientation between at least two bodies (3, 4, 13, 14, 16) is determined in a first measurement, at least one body (3, 4, 13, 14, 16) is processed and at least the spatial position and/or spatial orientation between at least two bodies (3, 4, 13, 14, 16) is determined in a second measurement.

19. The method according to any one of claims 12 to 17, ***characterized in that*** one body is a tooth or other bony body part, an implant or a template.

## Revendications

1. Dispositif pour la détermination et la mesure sans contact d'une position dans l'espace et/ou d'une orientation dans l'espace de corps, avec des corps (3, 4) tels qu'un outil médical, une pièce et/ou un instrument médical, et avec un système de suivi au moyen duquel les corps sont localisés et mis en relation les uns avec les autres, le système de suivi, ou au moins des éléments ou groupes d'éléments de celui-ci, pouvant être utilisé(s) de manière mobile, le dispositif présentant des moyens pour calculer un déplacement du système de suivi (2) mobile par rapport aux corps (3, 4) et le système de suivi présentant au moins deux caméras (5, 6), une optique et un système de capteurs, et des motifs (12, 15) sous la forme de complexes sensiblement bidimensionnels étant disposés sur les corps (3, 4), lesquels sont déterminés par le système de suivi mobile.

2. Dispositif selon la revendication 1, ***caractérisé en ce que*** les motifs se composent d'une combinaison de matériaux réfléchissants, absorbants, fluorescents ou auto-lumineux.

3. Dispositif selon l'une des revendications précédentes, ***caractérisé en ce que*** les motifs sont une couche colorée appliquée sur une structure.

4. Dispositif selon l'une des revendications précédentes, ***caractérisé en ce que*** le système de suivi (2) mobile présente un poids de moins de 0,1 kg.

5. Dispositif selon l'une des revendications précédentes, ***caractérisé en ce que*** le système de suivi (2) mobile présente un dispositif de fixation pour l'agencement sur un moyen de guidage déplaçable, tel un bras de support déplaçable manuellement ou un bras de robot.

6. Dispositif selon l'une des revendications précédentes, ***caractérisé en ce que*** le système de suivi (2) mobile présente une poignée (9).

7. Dispositif selon l'une des revendications précédentes, ***caractérisé en ce que*** les moyens de calcul sont réalisés en tant que logiciel.

8. Dispositif selon l'une des revendications précédentes, ***caractérisé en ce que*** le système de suivi (2) mobile présente une source d'énergie propre.

9. Dispositif selon l'une des revendications précédentes, ***caractérisé en ce que*** le système de suivi (2) mobile présente des moyens pour la communication sans fil.

10. Dispositif selon l'une des revendications précédentes, ***caractérisé en ce que*** le système de suivi (2) mobile présente des moyens pour l'éclairage (7, 8).

11. Dispositif selon l'une des revendications précédentes, ***caractérisé en ce que*** le système de suivi (2) mobile est disposé sur un outil ou un instrument médical.

12. Procédé pour la détermination et la mesure sans contact d'une position dans l'espace et/ou d'une orientation dans l'espace de corps avec un dispositif selon l'une des revendications précédentes, ***caractérisé en ce que*** le système de suivi mobile est bougé et déplacé par rapport à des corps pendant son utilisation, de manière manuelle ou de manière automatisée en étant monté sur un bras de robot.

13. Procédé selon la revendication 12, ***caractérisé en ce que*** l'on utilise, ensemble avec le système de suivi (2) mobile, un scanner optique mobile ou un projecteur mobile pour de la lumière structurée.

14. Procédé selon l'une des revendications 12 ou 13, ***caractérisé en ce que*** le système de suivi (2) mobile est tenu, pendant son utilisation, en particulier à la main, directement devant les zones de travail locales à détecter.

15. Procédé selon l'une des revendications 12 à 14, ***caractérisé en ce qu'**au* moins la position dans l'espace et/ou l'orientation dans l'espace entre au moins deux corps (4, 13, 14, 16) est déterminée simplement par phases.

16. Procédé selon l'une des revendications 12 à 15, ***caractérisé en ce que*** l'on détermine et mesure une géométrie d'un corps (4, 13, 14, 16).

17. Procédé selon l'une des revendications 12 à 15, ***caractérisé en ce que*** les corps (3, 4, 13, 14, 16) sont enregistrés au moyen de motifs (12, 15) se trouvant sur/au niveau des corps (3, 4, 13, 14, 16) et/ou de motifs secondaires, lesquels sont reconnus par d'autres systèmes d'imagerie.

18. Procédé selon l'une des revendications 12 à 16, ***caractérisé en ce que*** l'on détermine au moins la position dans l'espace et/ou l'orientation dans l'espace entre au moins deux corps (3, 4, 13, 14, 16) lors d'une première mesure, au moins un corps (3, 4, 13, 14, 16) étant traité et au moins la position dans l'espace et/ou l'orientation dans l'espace entre au moins deux corps (3, 4, 13 , 14, 16) étant déterminée lors d'une autre mesure.

19. Procédé selon l'une des revendications 12 à 17, ***caractérisé en ce qu'**un* corps est une dent, une autre partie de corps osseuse, un implant ou un gabarit.
